(19) Europäisches Patentamt
European Patent Office
Office européen des brevets

(11) EP 1 772 447 A1

(12) EUROPÄISCHE PATENTANMELDUNG

(43) Veröffentlichungstag:
**11.04.2007 Patentblatt 2007/15**

(51) Int Cl.:
***C07C 251/08*** *(2006.01)* ***C07D 265/06*** *(2006.01)*
***C07D 277/04*** *(2006.01)*

(21) Anmeldenummer: **05109110.6**

(22) Anmeldetag: **30.09.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Sika Technology AG**
**6340 Baar (CH)**

(72) Erfinder: **Burckhardt, Urs**
**8057, Zürich (CH)**

(74) Vertreter: **Isler, Jörg et al**
**c/o Sika Technology AG,**
**Tüffenwies 16-22**
**8048 Zürich (CH)**

(54) **Aldimine mit aktivem Wasserstoff aufweisenden Reaktivgruppen sowie deren Verwendung**

(57) Die vorliegende Erfindung betrifft Aldimine der Formel (I), deren Folgeprodukte sowie deren Verwendungen. Die Aldimine und Aldimin-haltigen Verbindungen zeichnen sich dadurch aus, dass sie geruchsfrei sind und bei der Hydrolyse geruchsfreie Aldehyde abspalten. Sie dienen deshalb als Quelle für Aldehyde und Amine.

EP 1 772 447 A1

## Beschreibung

### Technisches Gebiet

Die Erfindung betrifft das Gebiet der Aldimine.

### Stand der Technik

Aldimine sind Kondensationsprodukte aus Aminen und Aldehyden und stellen eine seit langem bekannte Stoffklasse dar. Bei Kontakt mit Wasser können Aldimine zu den entsprechenden Aminen und Aldehyden hydrolysieren, während sie in Abwesenheit von Wasser stabil sind. Aufgrund dieser Eigenart können sie als gebundene oder geschützte Form von Aminen, beziehungsweise Aldehyden, verwendet werden. So werden Aldimine beispielsweise in der Polyurethanchemie eingesetzt, wo sie als durch Feuchtigkeit aktivierbare Vernetzer, sogenannte "latente Amine" oder "latente Härter", für Isocyanat-haltige Kunststoffvorläufer dienen. Die Verwendung eines Aldimins als latenten Härter in Isocyanat-haltigen Systemen hat dabei zweierlei Vorteile: zum einen lässt sich die Entstehung von unerwünschten Gasblasen im ausgehärteten Kunststoff vermeiden, da die Aushärtung über das latente Amin - im Gegensatz zur direkten Reaktion des Isocyanats mit Feuchtigkeit - nicht unter Freisetzung von Kohlendioxid ($CO_2$) verläuft; zum anderen lassen sich hohe Aushärtungsgeschwindigkeiten erzielen. Jedoch birgt die Verwendung eines Aldimins in einem lagerfähigen Isocyanat-haltigen Kunststoffvorläufer die Gefahr, dessen Lagerstabilität durch vorzeitige Reaktion zwischen Aldimino- und Isocyanatgruppen herabzusetzen. Beispielsweise werden in US 4,469,831, US 4,853,454 und US 5,087,661 Zusammensetzungen aus Polyisocyanaten und Polyaldiminen beschrieben, welche unter dem Einfluss von Feuchtigkeit zu hochmolekularen Kunststoffen vernetzen und damit aushärten. Derartige Polyaldimine spalten bei der Hydrolyse jedoch stark riechende Aldehyde ab. WO 2004/013088 A1 beschreibt geruchsfreie Polyaldimine, welche aus der Reaktion von primären Polyaminen und geruchsfreien Aldehyden hergestellt werden.

Aldimine, welche zusätzliche funktionelle Gruppen aufweisen, sind bekannt. US 4,224,417 beschreibt beispielsweise Hydroxyaldimine und ihre Umsetzungsprodukte mit Polyisocyanaten. US 3,493,543, US 3,554,974, US 4,108,842, US 4,404,379 und US 6,136,942 beschreiben Aminoaldimine, beziehungsweise Cycloaminale als tautomere Form davon, ihre Umsetzungsprodukte mit Polyisocyanaten und deren Verwendung als latente Härter für Isocyanat-haltige Zusammensetzungen, die unter dem Einfluss von Feuchtigkeit rasch und blasenfrei aushärten. Die in den genannten Schriften beschriebenen Zusammensetzungen haben aber den Nachteil, eine stark eingeschränkte Lagerstabilität zu besitzen. Dies rührt daher, dass die geschützten Aminogruppen, welche in Form von Aldimino- oder Cycloaminal-Gruppen in den beschriebenen Aldiminen oder ihren Umsetzungsprodukten enthalten sind, sich gegenüber Isocyanatgruppen nicht vollständig inert verhalten, sondern mit diesen, insbesondere den reaktiven aromatischen Isocyanatgruppen, auch in Abwesenheit von Feuchtigkeit schleichend reagieren und dadurch eine Viskositätserhöhung verursachen, welche die Zusammensetzung schon nach kurzer Zeit unbrauchbar machen kann. Ein weiterer Nachteil der beschriebenen Aldimine enthaltend einen aktiven Wasserstoff, sowie deren Umsetzungsprodukte und Zusammensetzungen daraus, besteht darin, dass sie bei Kontakt mit Feuchtigkeit, bedingt durch die bei der Hydrolyse der Aldiminogruppen freigesetzten, geruchsintensiven Aldehyde, eine starke Geruchsbildung zeigen und deshalb, besonders in Innenräumen, nur eingeschränkt anwendbar sind.

### Darstellung der Erfindung

Aufgabe der vorliegenden Erfindung ist es daher, Aldimine zur Verfügung zu stellen, die geruchsfrei sind, Aldehyde abspalten, welche ebenfalls geruchsfrei sind, und sich insbesondere für Isocyanatgruppen aufweisende Kunststoffvorläufer einsetzen lassen, welche sich durch eine verbesserte Lagerstabilität auszeichnen.
Überraschenderweise hat sich gezeigt, dass Aldimine gemäss Anspruch 1 und 7 diese Aufgabe lösen. Es hat sich weiterhin gezeigt, dass mit Hilfe derartiger Aldimine eine breite Palette von Aldimin-haltigen Verbindungen gemäss Anspruch 8 erhältlich sind, welche über ausserordentliche Eigenschaften verfügen und welche sich als Kunststoffvorläufer oder als Bestandteil eines Kunststoffvorläufers einsetzen lassen. Isocyanat-haltige Zusammensetzungen, die unter Verwendung dieser Aldimin-haltigen Verbindungen hergestellt wurden, weisen eine gute Lagerstabilität auf. Solche Zusammensetzungen härten unter dem Einfluss von Feuchtigkeit rasch und ohne Blasenbildung aus, sind geruchsfrei und sind beispielsweise als Klebstoffe, Dichtstoffe, Beschichtungen oder Beläge mit guten mechanischen Eigenschaften geeignet. Weiterhin lassen sich die Aldimine der Formel (I) sowie die Aldimin-haltigen Verbindungen als Härter für zweikomponentige Isocyanat-haltige Zusammensetzungen verwenden, welche rasch, blasenfrei und ohne Geruchsbildung aushärten und beispielsweise als Klebstoffe, Dichtstoffe, Beschichtungen oder Beläge geeignet sind.

**Wege zur Ausführung der Erfindung**

Gegenstand der Erfindung sind Aldimine der Formel (I),

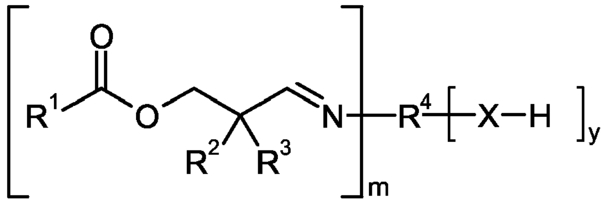

(I).

Hierbei steht m für eine ganze Zahl von 1 bis 4 und y für eine ganze Zahl von 1 bis 4, mit der Massgabe, dass die Summe von m und y einen Wert von 2 bis 5 darstellt. Weiterhin steht der Substituent $R^1$ entweder für einen einwertigen Kohlenwasserstoffrest mit 6 bis 30 C-Atomen, welcher gegebenenfalls mindestens ein Heteroatom, insbesondere in Form von Ether-Sauerstoff, aufweist, oder $R^1$ steht für einen Substituenten der Formel (II).

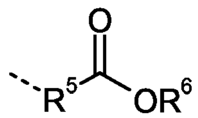

(II)

Hierbei steht der Substituent $R^5$ für einen zweiwertigen Kohlenwasserstoffrest mit 2 bis 20 C-Atomen, welcher gegebenenfalls mindestens ein Heteroatom, insbesondere in Form von Ether-Sauerstoff, aufweist. Der Substituent $R^6$ steht für einen einwertigen Kohlenwasserstoffrest mit 1 bis 20 C-Atomen.

Weiterhin stehen $R^2$ und $R^3$ entweder unabhängigfür zwei voneinander, je für unabhängige Substituenten, die jeweils einen einwertigen Kohlenwasserstoffrest mit 1 bis 12 C-Atomen darstellen, oder aber sie bilden zusammen einen einzigen Substituenten, der einen zweiwertigen Kohlenwasserstoffrest mit 4 bis 20 C-Atomen darstellt, welcher Teil eines carbocyclischen Rings mit 5 bis 8, bevorzugt 6, C-Atomen, wobei dieser ist carbocyclische Ring gegebenenfalls substituiert ist.

Weiterhin steht der Substituent $R^4$ für einen (m+y)-wertigen Kohlenwasserstoffrest mit 2 bis 12 C-Atomen, welcher gegebenenfalls mindestens ein Heteroatom, insbesondere in Form von Ether-Sauerstoff oder tertiärem Amin-Stickstoff, enthält.

Des Weiteren steht X für O, S oder N-$R^7$, wobei hierbei $R^7$ entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureestergruppe aufweist, steht, oder für einen Substituenten der Formel (III) steht.

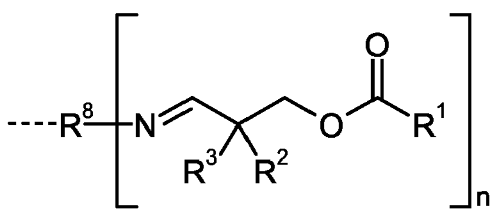

(III),

Hierbei stehen $R^8$ für einen (n+1)-wertigen Kohlenwasserstoffrest, welcher gegebenenfalls Heteroatome, insbesondere in Form von Ether-Sauerstoff oder tertiärem Amin-Stickstoff, und gegebenenfalls aktiven Wasserstoff in Form von Hydroxylgruppen, sekundären Aminogruppen oder Mercaptogruppen enthält, und n für eine ganze Zahl von 1 bis 10'000 steht. Die gestrichelten Linien in den Formeln dieses Dokumentes bezeichnen jeweils die Verbindungsstellen. In einer bevorzugten Ausführungsform ist y =1.

Das Aldimin der Formel (I) ist herstellbar aus mindestens einem sterisch gehinderten aliphatischen Aldehyd **A** und mindestens einem aliphatischen Amin **B**, entsprechend der Formel $[H_2N]_m$-$R^4$-$[XH]_y$, welches neben einer oder mehreren primären Aminogruppen noch mindestens eine weitere Reaktivgruppe enthaltend einen aktiven Wasserstoff aufweist. Der Begriff "aktiver Wasserstoff" bezeichnet im vorliegenden Dokument ein deprotonierbares, an ein Stickstoff-, Sauerstoff- oder Schwefelatom gebundenes Wasserstoffatom. Der Begriff "Reaktivgruppe enthaltend einen aktiven

Wasserstoff' bezeichnet eine einen aktiven Wasserstoff aufweisende funktionelle Gruppe, insbesondere eine primäre oder sekundäre Aminogruppe, eine Hydroxylgruppe, eine Mercaptogruppe oder eine Harnstoffgruppe.

Die Umsetzung zwischen dem Aldehyd **A** und dem Amin **B** erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **A** wird hierbei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (I) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Für die Herstellung des Aldimins der Formel (I) wird mindestens ein sterisch gehinderter aliphatischer Aldehyd **A** der Formel (IV) eingesetzt.

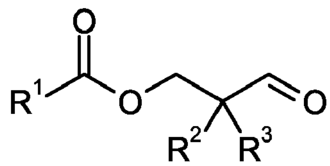

(IV)

In der Formel (IV) haben $R^1$, $R^2$ und $R^3$ die gleiche Bedeutung wie für Formel (I) beschrieben.

Der Aldehyd **A** ist geruchsfrei. Unter einer "geruchsfreien" Substanz wird eine Substanz verstanden, die so geruchsarm ist, dass sie für die meisten menschlichen Individuen nicht riechbar, das heisst mit der Nase nicht wahrnehmbar, ist.

Der Aldehyd **A** wird beispielsweise hergestellt aus einer Carbonsäure $R^1$-COOH und einem ß-Hydroxyaldehyd der Formel (V) in einer Veresterungsreaktion. Diese Veresterung kann nach bekannten Methoden erfolgen, beschrieben beispielsweise in Houben-Weyl, "Methoden der organischen Chemie", Vol. VIII, Seiten 516-528. Der ß-Hydroxyaldehyd der Formel (V) wird beispielsweise in einer gekreuzten Aldol-Addition aus Formaldehyd (oder oligomeren Formen von Formaldehyd, wie Paraformaldehyd oder 1,3,5-Trioxan) und einem Aldehyd der Formel (VI) erhalten.

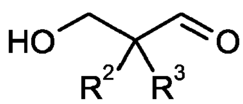

(V)

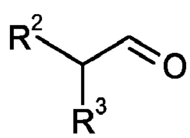

(VI)

In den Formeln (V) und (VI) haben $R^2$ und $R^3$ die gleiche Bedeutung wie für Formel (I) beschrieben.

Die Herstellung des Aldehyds **A** erfolgt bevorzugt lösemittelfrei. Dabei wird der ß-Hydroxyaldehyd der Formel (V) ohne Verwendung von Lösemitteln direkt mit der Carbonsäure umgesetzt, wobei das bei der Veresterung gebildete Wasser im Vakuum entfernt wird. Es ist weiterhin bevorzugt, die zum Aldehyd **A** führenden Aldol- und Veresterungsreaktionen aus den Grundstoffen in einem gemeinsamen Prozessschritt, als Eintopfreaktion, auszuführen.

Als geeignete Carbonsäuren $R^1$-COOH zur Veresterung mit den ß-Hydroxyaldehyden der Formel (V) seien beispielsweise die folgenden erwähnt: gesättigte aliphatische Carbonsäuren wie Oenanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure; einfach ungesättigte aliphatische Carbonsäuren wie Palmitoleinsäure, Ölsäure, Erucasäure; mehrfach ungesättigte aliphatische Carbonsäuren wie Linolsäure, Linolensäure, Elaeostearinsäure, Arachidonsäure; cycloaliphatische Carbonsäuren wie Cyclohexancarbonsäure; arylaliphatische Carbonsäuren wie Phenylessigsäure; aromatische Carbonsäuren wie Benzoesäure, Naphthoesäure, Toluylsäure, Anissäure; Isomere dieser Säuren; Fettsäuregemische aus der technischen Verseifung von natürlichen Ölen und Fetten wie

beispielsweise Rapsöl, Sonnenblumenöl, Leinöl, Ölbaumöl, Kokosnussöl, Ölpalmkernöl und Ölpalmöl; sowie Dicarbonsäuremonoalkyl- und -arylester, wie sie aus der einfachen Veresterung von Dicarbonsäuren wie Bernsteinsäure, Glutarsäure, Adipinsäure, Pimelinsäure, Korksäure, Azelainsäure, Sebacinsäure, 1,12-Dodecandisäure, Maleinsäure, Fumarsäure, Hexahydrophthalsäure, Hexahydroisophthalsäure, Hexahydroterephthalsäure, 3,6,9-Trioxaundecandisäure und ähnliche Derivate von Polyethylenglykol, mit Alkoholen wie Methanol, Ethanol, Propanol, Butanol, höheren Homologen und Isomeren dieser Alkohole erhalten werden.
Bevorzugt sind Caprylsäure, Caprinsäure, Laurinsäure, Myristinsäure, Palmitinsäure, Stearinsäure, Ölsäure, Linolsäure, Linolensäure, die Isomeren dieser Säuren sowie technische Gemische von Fettsäuren, welche diese Säuren enthalten. Besonders bevorzugt ist Laurinsäure.

Geeignete Aldehyde der Formel (VI) zur Umsetzung mit Formaldehyd zu ß-Hydroxyaldehyden der Formel (V) sind beispielsweise Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.
Geeignete ß-Hydroxyaldehyde der Formel (V) sind beispielsweise die Produkte aus der Umsetzung von Formaldehyd mit den vorgängig als geeignet genannten Aldehyden der Formel (VI). Bevorzugt ist 3-Hydroxypivalaldehyd.

Das Amin **B** ist ein aliphatisches Amin, welches neben einer oder mehreren primären Aminogruppen noch mindestens eine weitere Reaktivgruppe, welche einen aktiven Wasserstoff enthält, aufweist. Der Begriff "primäre Aminogruppe" bezeichnet im vorliegenden Dokument eine $NH_2$-Gruppe, die an einen organischen Rest gebunden ist, während der Begriff "sekundäre Aminogruppe" eine NH-Gruppe bezeichnet, die an zwei organische Reste gebunden ist. Der Begriff "aliphatisches Amin" bezeichnet Verbindungen, die mindestens eine Aminogruppe enthalten, die an einen aliphatischen, cycloaliphatischen oder arylaliphatischen Rest gebunden ist. Sie unterscheiden sich damit von den aromatischen Aminen, in welchen die Aminogruppe direkt an einen aromatischen Rest gebunden ist, wie beispielsweise in Anilin oder 2-Aminopyridin.

Das Amin **B** enthält neben einer oder mehreren primären Aminogruppen eine oder mehrere weitere Reaktivgruppen, welche einen aktiven Wasserstoff enthalten.
In einer Ausführungsform enthält das Amin **B** nur eine weiter derartige Reaktivgruppe.
Als Amine **B,** welche neben einer oder mehreren primären Aminogruppen nur eine weitere Reaktivgruppe, welche einen aktiven Wasserstoff enthält, aufweisen, eignen sich beispielsweise die im Folgenden genannten Verbindungen:

- aliphatische Hydroxyamine wie 2-Aminoethanol, 2-Methylaminoethanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykol-monoamin, $\alpha$-(2-Hydroxymethylethyl)-$\omega$-(2-aminomethyl-ethoxy)-poly(oxy(methyl-1,2-ethandiyl)); eine Hydroxylgruppe und eine oder mehrere primäre Aminogruppen tragende Derivate von polyalkoxylierten drei-oder höherwertigen Alkoholen oder von polyalkoxylierten Diaminen; Produkte aus der einfachen Cyanoethylierung und anschliessender Hydrierung von Glykolen, beispielsweise 3-(2-Hydroxyethoxy)-propylamin, 3-(2-(2-Hydroxy-ethoxy)-ethoxy)-propylamin, 3-(6-Hydroxyhexyloxy)-propylamin;
- aliphatische Mercaptoamine wie 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dodecanthiol; Aminothiozucker wie 2-Amino-2-deoxy-6-thioglucose;
- zwei- oder mehrwertige aliphatische Amine, welche neben einer oder mehreren primären Aminogruppen eine sekundäre Aminogruppe tragen, wie N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-Aminoethyl-piperazin, Diethylentriamin (DETA), Bis-hexamethylentriamin (BHMT); Di-und Triamine aus der Cyanoethylierung oder Cyanobutylierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin,3-Ethylamino-1-pentylamin,3-Butyl-amino-1-pentylamin, 3-Hexylamino-1-pentylamin, 3-(2-Ethylhexyl)amino-1-pentylamin,3-Dodecylamino-1-pentylamin,3-Cyclohexylamino-1-pentylamin, Dipropylentriamin (DPTA), N3-(3-Aminopentyl)-1,3-pentandiamin, N5-(3-Amino-propyl)-2-methyl-1,5-pentandiamin, N5-(3-Amino-1-ethylpropyl)-2-methyl-1,5-pentandiamin,und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-($C_{16-22}$-Alkyl)-1,3-propandiamin, wie sie beispielsweise unter dem Handelsnamen Duomeen® von Akzo Nobel erhältlich sind; die Produkte aus der Michael-artigen Addition von aliphatischen primären Di- oder Polyaminen mit Acrylnitril, Malein- oder Fumarsäurediestern, Citraconsäurediestern, Acryl- und Methacrylsäureestern und Itaconsäurediestern, umgesetzt im Molverhältnis 1:1;

- trisubstituierte Harnstoffe, welche eine oder mehrere primäre Aminogruppen tragen, wie N-(2-Aminoethyl)-ethylenharnstoff, N-(2-Aminoethyl)-propylenharnstoff oder N-(2-Aminoethyl)-N'-methylharnstoff. Insbesondere geeignete aliphatische Hydroxy- und Mercaptoamine sind solche, in denen die primäre Aminogruppe von der Hydroxyl- beziehungsweise der Mercaptogruppe durch eine Kette von mindestens 5 Atomen, oder durch einen Ring, getrennt sind, wie beispielsweise in 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethylcyclohexanol, 2-(2-Aminoethoxy)-ethanol, Triethylenglykol-monoamin, $\alpha$-(2-Hydroxymethylethyl)-$\omega$-(2-aminomethylethoxy)-poly(oxy(methyl-1,2-ethandiyl)), 3-(2-Hydroxyethoxy)-propylamin, 3-(2-(2-Hydroxyethoxy)-ethoxy)-propylamin, 3-(6-Hydroxyhexyloxy)-propylamin, 6-Amino-1-hexanthiol, 8-Amino-1-octanthiol, 10-Amino-1-decanthiol und 12-Amino-1-dodecanthiol.

Als Amine **B,** welche neben einer oder mehreren primären Aminogruppen nur eine weitere Reaktivgruppe enthaltend einen aktiven Wasserstoff aufweisen, bevorzugt sind zwei- oder mehrwertige aliphatische Amine, welche neben einer oder mehreren primären Aminogruppen eine sekundäre Aminogruppe tragen, insbesondere N-Methyl-1,2-ethandiamin, N-Ethyl-1,2-ethan-diamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin. Bevorzugt sind auch aliphatische Hydroxy- und Mercaptoamine, in denen die primäre Aminogruppe von der Hydroxy- beziehungsweise der Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxyethoxy)-propylamin, 3-(2-(2-Hydroxyethoxy)-ethoxy)-propylamin sowie 3-(6-Hydroxyhexyloxy)-propylamin.

In einer weiteren Ausführungsform enthält das Amin **B** neben einer oder mehreren primären Aminogruppen weitere Reaktivgruppen, welche einen aktiven Wasserstoff enthalten.

Als Amine **B,** welche neben einer oder mehreren primären Aminogruppen mehrere weitere Reaktivgruppen enthaltend einen aktiven Wasserstoff aufweisen, eignen sich beispielsweise die im Folgenden genannten Verbindungen:

- zwei- oder mehrwertige aliphatische Amine, welche neben einer oder mehreren primären Aminogruppen mehr als eine sekundäre Aminogruppe tragen, wie Triethylentetramin (TETA), Tetraethylenpentamin (TEPA), Pentaethylenhexamin und höhere Homologe linearer Polyethylenamine, N,N'-Bis-(3-aminopropyl)-ethylendiamin, sowie Polyethylenimine unterschiedlichen Polymerisationsgrades (Molmassen-Bereich 500 bis 1'000'000 g/mol), wie sie beispielsweise unter dem Handelsnamen Lupasol® von BASF in reiner Form oder als wässrige Lösungen erhältlich sind, wobei diese Polyethylenimine neben primären und sekundären auch tertiäre Aminogruppen enthalten;
- mehr als eine Hydroxylgruppe und eine oder mehrere primäre Aminogruppen tragende Derivate von polyalkoxylierten drei- oder höherwertigen Alkoholen oder von polyalkoxylierten Polyaminen.

  Als Amine **B,** welche neben einer oder mehreren primären Aminogruppen mehrere weitere Reaktivgruppen enthaltend einen aktiven Wasserstoff aufweisen, bevorzugt sind zwei- oder mehrwertige aliphatische Amine, welche neben einer oder mehreren primären Aminogruppen mehr als eine sekundäre Aminogruppe tragen, wie Triethylentetramin, Tetraethylenpentamin, Pentaethylenhexamin und höhere Homologe linearer Polyethylenamine; Hydroxypolyamine wie N-Hydroxyethyl-1,2-ethandiamin, N-Hydroxypropyl-1,2-ethandiamin, N-Hydroxyethyl-1,3-propandiamin, N3-Hydroxyethyl-1,3-pentandiamin; Polyamine aus der mehrfachen Cyanoethylierung oder Cyanobutylierung von primären Di- und Polyaminen und von Hydroxyaminen mit mehreren primären Aminogruppen, wie N,N'-Bis-(3-aminopropyl)-ethylendiamin, N,N'-Bis-(3-aminopropyl)-1,4-diaminobutan, N,N'-Bis-(3-aminopropyl)-2-methyl-1,5-pentandiamin, N,N'-Bis-(3-amino-1-ethylpropyl)-2-methyl-1,5-pentandiamin; sowie verzweigte Polyethylenimine unterschiedlichen Polymerisationsgrades (Molmassen-Bereich 500 bis 1'000'000 g/mol).

  Die Umsetzung zwischen einem Aldehyd **A** und einem Amin **B** führt dabei zu Hydroxyaldiminen, wenn als Amin **B** ein Hydroxyamin eingesetzt wird; zu Mercaptoaldiminen, wenn als Amin **B** ein Mercaptoamin eingesetzt wird; zu Aminoaldiminen, wenn als Amin **B** ein zwei- oder mehrwertiges Amin, welches neben einer oder mehreren primären Aminogruppen eine oder mehrere sekundäre Aminogruppen trägt, eingesetzt wird; oder zu Harnstoffaldiminen, wenn als Amin **B** ein trisubstituierter Harnstoff, welcher eine oder mehrere primäre Aminogruppen trägt, eingesetzt wird.

  In einer Ausführungsform weisen die Aldimine der Formel (I) einen Substituenten N-$R^7$ als Substituenten X auf. Derartige Aldimine der Formel (I) lassen sich dadurch herstellen, dass mindestens ein sterisch gehinderter aliphatischer Aldehyd **A** der Formel (IV) mit mindestens einem zwei- oder mehrwertigen aliphatischen primären Amin **C** der Formel $[H_2N]_m$-$R^4$-$[NH_2]_y$ in einem ersten Schritt zu einem Zwischenprodukt der Formel (VII) umgesetzt wird, welches neben einer oder mehreren Aldiminogruppen noch mindestens eine, bevorzugt eine, primäre Aminogruppe enthält, und dieses Zwischenprodukt anschliessend in einem zweiten Schritt in einer Additionsreaktion mit einem

Michael-Akzeptor der Formel (VIII) in einem Verhältnis der Anzahl Doppelbindungen : Anzahl $NH_2$-Gruppen = 1:1 umgesetzt wird. Dabei entsteht ein Aminoaldimin, welches neben einer oder mehreren Aldiminogruppen noch mindestens eine, bevorzugt eine, sekundäre Aminogruppe enthält.

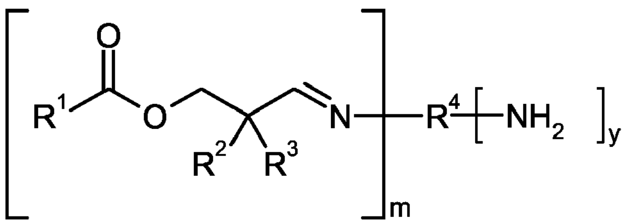 (VII)

In der Formel (VII) haben m, y, $R^1$, $R^2$, $R^3$ und $R^4$ die gleiche Bedeutung wie für Formel (I) beschrieben.

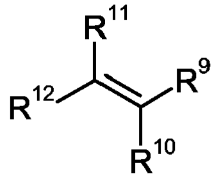 (VIII)

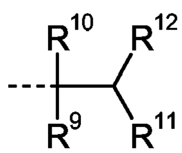 (IX)

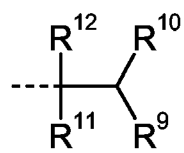 (IX')

Somit entstehen Aldimine der Formel (I), bei welchen X für den Rest N-$R^7$ steht, und $R^7$ ein einwertiger Kohlenwasserstoffrest der Formel (IX) oder (IX') darstellt. In den Formeln (VIII), (IX) und (IX') stehen hierbei $R^9$ für einen Rest, welcher ausgewählt ist aus der Gruppe bestehend aus $-COOR^{13}$, -CN, $-NO_2$, $-PO(OR^{13})_2$, $-SO_2R^{13}$ und $-SO_2OR^{13}$ und $R^{10}$ für ein Wasserstoffatom oder einen Rest aus der Gruppe bestehend aus $-R^{13}$, $-COOR^{13}$ und $-CH_2COOR^{13}$ und $R^{11}$ und $R^{12}$ unabhängig voneinander für ein Wasserstoffatom oder einen Rest aus der Gruppe bestehend aus $-R^{13}$, $-COOR^{13}$ und -CN, wobei $R^{13}$ für einen einwertigen Kohlenwasserstoffrest mit 1 bis 20 C-Atomen steht.

Das Amin **C** ist ein aliphatisches Amin mit mindestens zwei primären Aminogruppen. Der Begriff "aliphatisches primäres Amin" bezeichnet ein aliphatisches Amin, in welchem die Aminogruppe eine primäre Aminogruppe ist.

Beispiele für geeignete Amine **C** sind aliphatische Polyamine wie Ethylendiamin, 1,2- und 1,3-Propandiamin, 2-Methyl-1,2-propandiamin, 2,2-Dimethyl-1,3-propandiamin, 1,3- und 1,4-Butandiamin, 1,3- und 1,5-Pentandiamin, 2-Butyl-2-ethyl-1,5-pentandiamin, 1,6-Hexamethylendiamin (HMDA), 2,2,4- und 2,4,4-Trimethylhexamethylendiamin und Mischungen davon (TMD), 1,7-Heptandiamin, 1,8-Octandiamin, 2,4-Dimethyl-1,8-octandiamin, 4-Aminomethyl-1,8-octandiamin, 1,9-Nonandiamin, 2-Methyl-1,9-nonandiamin, 5-Methyl-1,9-nonandiamin, 1,10-Decandiamin, Isodecandiamin, 1,11-Undecandiamin, 1,12-Dodecandiamin, Methyl-bis-(3-aminopropyl)amin, 1,5-Diamino-2-methylpentan (MPMD), 1,3-Diaminopentan(DAMP), 2,5-Dimethyl-1,6-hexamethylendiamin, cycloaliphatische Polyamine wie 1,2-, 1,3- und 1,4-Diaminocyclohexan, Bis-(4-aminocyclohexyl)-methan ($H_{12}$MDA), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 1,3,5-Tris-(aminomethyl)-cyclohexan, 1-Cyclohexylamino-3-aminopropan, 2,5(2,6)-Bis-(aminomethyl)-bicyclo[2.2.1]heptan (NBDA, hergestellt von Mitsui Chemicals), 3(4),8(9)-Bis-(aminomethyl)-tricyclo[5.2.1.$0^{2,6}$]decan, 1,4-Diamino-2,2,6-trimethylcyclohexan (TMCDA), 3,9-Bis-(3-aminopropyl)-2,4,8,10-tetraoxaspiro[5.5]undecan, arylaliphatische Polyamine wie 1,3-Xylylendiamin (MXDA), 1,4-Xylylendiamin (PXDA), 1,3,5-Tris-(aminomethyl)benzol, Ethergruppen-haltige aliphatische Polyamine wie Bis-(2-aminoethyl)ether, 4,7-Dioxadecan-1,10-diamin, 4,9-Dioxadodecan-1,12-diamin und höhere Oligomere davon, Polyoxyalkylen-Polyamine mit theoretisch zwei oder drei Aminogruppen, erhältlich beispielsweise unter dem Namen Jeffamine® (hergestellt von Huntsman Chemicals). Bevorzugt sind Di-oder Triamine, in denen die primären Aminogruppen durch eine Kette von mindestens 5 Atomen, oder durch einen Ring, getrennt sind, insbesondere 1,5-Diamino-2-methylpentan, 1,6-Hexamethylendiamin, 2,2,4- und 2,4,4-Trimethylhexamethylendiamin und Mischungen davon, 1,10-Decandiamin, 1,12-Dodecandiamin, 1,3- und 1,4-Diaminocyclohexan, Bis-(4-aminocyclohexyl)-methan, Bis-(4-amino-3-methylcyclohexyl)-methan, 1-Amino-3-aminomethyl-3,5,5-trimethylcyclohexan, 1,3- und 1,4-Bis-(aminomethyl)cyclohexan, 2,5(2,6)-Bis-(aminomethyl)-bicyclo[2.2.1]heptan, 3(4),8(9)-Bis-(aminomethyl)-tricyclo-[5.2.1.$0^{2,6}$]decan, 1,4-Diamino-2,2,6-trimethylcyclohexan (TMCDA), 3,9-Bis-(3-aminopropyl)-2,4,8,10-tetraoxaspiro[5.5]undecan, 1,3- und 1,4-Xylylendiamin, 1,3,5-Tris-(aminomethyl)benzol sowie Polyoxyalkylen-Polyamine mit theoretisch zwei oder drei Aminogruppen, erhältlich beispielsweise unter dem Namen Jeffamine® (hergestellt von Huntsman Chemicals).

Beispiele für geeignete Michael-Akzeptoren der Formel (VIII) sind Malein- oder Fumarsäurediester wie Dimethylmaleinat, Diethylmaleinat, Dibutylmaleinat, Diethylfumarat; Citraconsäurediester wie Dimethylcitraconat; Acryl- oder Methacrylsäureester wie Methyl(meth)acrylat, Ethyl(meth)acrylat, Butyl(meth)acrylat, Lauryl(meth)acrylat, Stearyl (meth)acrylat, Tetrahydrofuryl-(meth)acrylat, Isobornyl(meth)acrylat; Itaconsäurediester wie Dimethylitaconat; Zimtsäureester wie Methylcinnamat; Vinylphosphonsäurediester wie Vinylphosphonsäuredimethylester; Vinylsulfonsäureester, insbesondere Vinylsulfonsäurearylester; Vinylsulfone; Vinylnitrile wie Acrylnitril, 2-Pentennitril oder Fumaronitril; 1-Nitroethylene wie β-Nitrostyrol; und Knoevenagel-Kondensationsprodukte, wie beispielsweise solche aus Malonsäurediestern und Aldehyden wie Formaldehyd, Acetaldehyd oder Benzaldehyd. Bevorzugt sind Maleinsäurediester, Acrylsäureester, Phosphonsäurediester und Vinylnitrile.

Die Umsetzung des Aldehyds **A** mit dem Amin **C** zum Zwischenprodukt der Formel (VII) erfolgt in einer Kondensationsreaktion unter Abspaltung von Wasser, wie sie weiter oben für die Umsetzung des Aldehyds **A** mit dem Amin **B** beschrieben wird. Die Stöchiometrie zwischen dem Aldehyd **A** und dem Amin C wird dabei so gewählt, dass m mol Aldehyd **A** für 1 mol Amin **C,** welches y+m primäre Aminogruppen enthält, eingesetzt werden. Es wird ein lösemittelfreies Herstellverfahren bevorzugt, wobei das bei der Kondensation gebildete Wasser mittels Anlegen von Vakuum aus der Reaktionsmischung entfernt wird. Bevorzugt ist y=1.

Die Umsetzung des Zwischenprodukts der Formel (VII) mit dem Michael-Akzeptor der Formel (VIII) erfolgt beispielsweise dadurch, dass das Zwischenprodukt mit einer stöchiometrischen oder leicht überstöchiometrischen Menge des Michael-Akzeptors der Formel (VIII) gemischt wird und die Mischung bei Temperaturen von 20 bis 110°C bis zum vollständigen Umsatz des Zwischenprodukts zum Aldimin der Formel (I) erwärmt wird. Die Umsetzung erfolgt bevorzugt ohne eine Verwendung von Lösemitteln.

Die Aldimine der Formel (I) können gegebenenfalls im Gleichgewicht stehen mit cyclischen Formen, wie sie in Formel (X) beispielhaft gezeigt sind. Diese cyclischen Formen sind im Fall von Aminoaldiminen cyclische Aminale, beispielsweise Imidazolidine oder Tetrahydropyrimidine; im Fall von Hydroxyaldiminen cyclische Aminoacetale, beispielsweise Oxazolidine oder Tetrahydrooxazine; im Fall von Mercaptoaldiminen cyclische Thioaminale, beispielsweise Thiazolidine oder Tetrahydrothiazine.

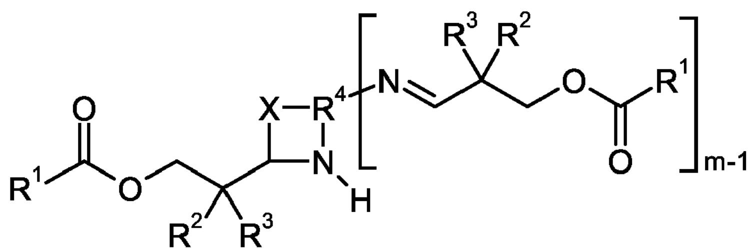
(X)

In der Formel (X) haben m, $R^1$, $R^2$, $R^3$, $R^4$ und X die gleiche Bedeutung wie für Formel (I) beschrieben.

Überraschenderweise neigen die meisten Aldimine der Formel (I) nicht zur Cyclisierung. Insbesondere für Aminoaldimine kann mittels IR- und NMRspektroskopischen Methoden gezeigt werden, dass diese Verbindungen überwiegend in der offenkettigen, also der Aldimin-Form, vorliegen, während die cyclische, also die Aminal-Form, nicht oder nur in Spuren vorkommt. Dies steht im Gegensatz zum Verhalten der Aminoaldimine nach dem Stand der Technik, wie sie beispielsweise in US 4,404,379 und US 6,136,942 beschrieben werden: jene liegen nämlich hauptsächlich in der Cycloaminal-Form vor. Auch Hydroxy- und Mercaptoamine, in denen die primäre Aminogruppe von der Hydroxy- beziehungsweise der Mercaptogruppe durch eine Kette von mindestens 5 Atomen, oder durch einen Ring, getrennt sind, zeigen kaum Cyclisierung. Die weitgehende Abwesenheit cyclischer Strukturen in den Aldiminen der Formel (I) ist, insbesondere im Hinblick auf deren Verwendung in Isocyanat-haltigen Zusammensetzungen, als Vorteil zu werten, da die Aldimine dadurch weitgehend frei sind von den in Aminalen, Oxazolidinen und Thioaminalen vorkommenden basischen Stickstoffatomen, welche die Lagerstabilität der Isocyanat-haltigen Zusammensetzung herabsetzen könnten.

Die Aldimine der Formel (I) sind geruchsfrei. Sie sind unter geeigneten Bedingungen, insbesondere unter Ausschluss

von Feuchtigkeit, lagerstabil. Bei Zutritt von Feuchtigkeit können die Aldiminogruppen der Aldimine über Zwischenstufen formal zu Aminogruppen hydrolysieren, wobei der entsprechende, zur Herstellung des Aldimins verwendete Aldehyd **A** freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen teilweise oder ganz hydrolysieren.

Die Aldimine der Formel (I) lassen sich sehr breit verwenden. Grundsätzlich lassen sie sich überall dort einsetzen, wo sie als Quelle der Aldehyde der Formel (IV) oder der Amine **B** dienen können. Insbesondere können sie in der Funktion von geschützten Aminen, beziehungsweise geschützten Aldehyden, in Aldehyd- und/oder oder Amin-reaktiven Systemen eingesetzt werden können und dort bei Bedarf gezielt entschützt werden. Insbesondere finden sie in Systemen Anwendungen, in welchen Verbindungen vorhanden sind, welche mit primären Aminen reagieren. Die Entschützung erfolgt hydrolytisch, beispielsweise durch Kontakt mit Wasser oder Feuchtigkeit, insbesondere Luftfeuchtigkeit.

Andererseits finden die Aldimine der Formel (I) Verwendung beim Aufbau von weiter funktionalisierten Reaktionsprodukten der Aldimine der Formel (I). So können Aldimine der Formel (I) mit Verbindungen umgesetzt werden, welche mit der Gruppe XH reagieren. In der Folge lassen sich diese Reaktionsprodukte bei Bedarf zu Aldehyden der Formel (IV) und Verbindungen mit primären Aminogruppen hydrolysieren, was dann Anlass zu Reaktionen oder Vernetzungen gibt. Durch Reduktion der Aldiminogruppen können die Aldimine der Formel (I) auch zum Aufbau von Verbindungen enthaltend spezielle sekundäre Aminogruppen dienen, welche beispielsweise als Härter für Isocyanat-und/oder Epoxid-haltigen Zusammensetzungen eingesetzt werden können.

Die Aldimine der Formel (I) lassen sich beispielsweise als Bausteine für Kunststoffvorläufer verwenden. Mit dem Begriff "Kunststoffvorläufer" werden im vorliegenden Dokument monomere, oligomere oder polymere organische Verbindungen - oder solche Verbindungen zu einem wesentlichen Teil enthaltende homogene oder heterogene Zusammensetzungen - bezeichnet, welche aufgrund von in ihnen enthaltenen, für Polyreaktionen zugänglichen Reaktivgruppen befähigt sind, mit sich allein oder zusammen mit anderen Molekülen zu hochmolekularen Kunststoffen, das heisst organischen Polymeren, auszureagieren, ein Prozess, der gemeinhin als "Aushärtung" oder auch als "Vernetzung" bezeichnet wird - unabhängig davon, ob die bei der Aushärtung ablaufenden Reaktionen zu kovalent oder andersartig vernetzten Strukturen führen. Der Begriff "Polyreaktionen" umfasst dabei sämtliche Arten von Polyadditions-, Polykondensation- und Polymerisationsreaktionen. Der Begriff "Polymer" umfasst im vorliegenden Dokument sowohl ein Kollektiv von chemisch einheitlichen, sich aber in Bezug auf Polymerisationsgrad, Molmasse und Kettenlänge unterscheidenden Makromolekülen, die durch eine Polyreaktion hergestellt wurden, als auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Die Vorsilbe "Poly" in Substanzbezeichnungen, wie "Polyaldimin", "Polyamin", "Polyisocyanat" oder "Polyol" weist im vorliegenden Dokument darauf hin, dass die jeweilige Substanz formal mehr als eine der in ihrer Bezeichnung vorkommenden funktionellen Gruppe pro Molekül enthält. Attribute von Substanzen wie "Aldimin-haltig" oder "Isocyanathaltig" weisen darauf hin, dass die bezeichneten funktionellen Gruppen, also Aldiminogruppen oder Isocyanatgruppen, in den Substanzen enthalten sind.

In einer ersten bevorzugten Verwendungsart werden Aldimine der Formel (I) zur Herstellung von Aldimin-haltigen Verbindungen eingesetzt, welche beispielsweise als latente Härter oder als Co-Monomere für Kunststoffvorläufer, insbesondere für Isocyanat-haltige Zusammensetzungen, geeignet sind. Ein weiterer Gegenstand der vorliegenden Erfindung stellen Aldimin-haltige Verbindungen **AV** dar, welche Additionsprodukte darstellen aus der Umsetzung von mindestens einem Aldimin der Formel (I) mit y=1 mit mindestens einer Verbindung **D,** die mehr als eine Reaktivgruppe trägt, welche mit der Gruppe XH Additionsreaktionen eingehen kann. Die den aktiven Wasserstoff tragende Reaktivgruppe des Aldimins der Formel (I) reagiert dabei in einer Additionsreaktion mit einer oder mehreren Reaktivgruppen der Verbindung **D** zu einer im Folgenden auch "Additionsprodukt" genannten Aldimin-haltigen Verbindung **AV.**

Wird die Umsetzung stöchiometrisch geführt, das heisst mit einem Molequivalent aktiven Wasserstoff des Aldimins der Formel (I) auf ein Molequivalent Reaktivgruppen der Verbindung **D** - wodurch deren Reaktivgruppen vollständig umgesetzt werden -, wird als Aldimin-haltige verbindung **AV** ein Polyaldimin erhalten. Auf einfache Weise werden so vielfältige Polyaldimine erhalten, ohne dass zu ihrer Herstellung auf die entsprechenden primären Polyamine, welche technisch und kommerziell nur beschränkt zur Verfügung stehen, zurückgegriffen werden müsste. In Abhängigkeit von Struktur, Funktionalität und Molekulargewicht der Verbindungen D und der Aldimine der Formel (I) können diese Polyaldimine höchst unterschiedliche Eigenschaften aufweisen; sie lassen sich also auf die Bedürfnisse einer bestimmten Anwendung massschneidern. Diese Polyaldimine eignen sich insbesondere als latente Härter für Isocyanat-haltige Zusammensetzungen.

Durch geeignete Umsetzung der Aldimine der Formel (I) mit Verbindungen D können auch heterofunktionelle Additionsprodukte hergestellt werden, das heisst, solche Aldimin-haltige Verbindungen **AV,** welche neben einer oder

mehreren Aldiminogruppen noch eine oder mehrere andere, für Polyreaktionen zugängliche Reaktivgruppen aufweisen. Heterofunktionelle Additionsprodukte werden dann erhalten, wenn die Umsetzung zwischen dem Aldimin und einer Verbindung **D** unterstöchiometrisch, das heisst mit weniger als einem Molequivalent aktiven Wasserstoff des Aldimins auf ein Molequivalent Reaktivgruppen der Verbindung **D,** geführt wird. Die Verbindung **D** selbst kann dabei homo- oder heterofunktionell sein. Solche heterofunktionellen Additionsprodukte sind beispielsweise verwendbar als Co-Monomere oder als latente Härter für Kunststoffvorläufer; oder, wenn die Aldiminogruppe als solche oder nach ihrer Hydrolyse mit den anderen im heterofunktionellen Additionsprodukt vorliegenden Reaktivgruppen unter Verknüpfung der Moleküle reagieren kann, auch als Kunststoffvorläufer selbst. Dies trifft insbesondere zu für den Fall von Aldimin-haltigen Verbindungen **AV,** die zusätzlich Isocyanatgruppen enthalten.

Als Verbindungen **D** geeignet sind Substanzen, welche mehr als eine der folgenden Reaktivgruppen, welche Additionsreaktionen eingehen können, tragen: Isocyanat-, Isothiocyanat-, Cyclocarbonat-, Epoxid-, Episulfid-, Aziridin-, Acryl-, Methacryl-, 1-Ethinylcarbonyl-, 1-Propinylcarbonyl-, Maleimid-, Citraconimid-, Vinyl-, Isopropenyl- und Allyl-Gruppen, sowie Verbindungen mit verschiedenen der vorgenannten Reaktivgruppen. Bevorzugt sind Isocyanat-, Epoxid-, Acryl-, Maleimid-, Vinyl-, Isopropenyl- und Allylgruppen. Besonders bevorzugt ist die Isocyanatgruppe.

Beispiele für geeignete Verbindungen **D** sind

- zwei- oder mehrwertige, mono- und/oder oligomere aliphatische, cycloaliphatische, arylaliphatische und aromatische Isocyanate (Polyisocyanate) wie 1,6-Hexamethylendiisocyanat (HDI), 2-Methylpentamethylen-1,5-diisocyanat, 2,2,4- und 2,4,4-Trimethyl-1,6-hexamethylendiisocyanat (TMDI), 1,12-Dodecamethylendiisocyanat, Lysin- und Lysinesterdiisocyanat, Cyclohexan-1,3- und -1,4-diisocyanat und beliebige Gemische dieser Isomeren, 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), 1,3,5-Tris-(isocyanatomethyl)-benzol, m- und p-Tetramethyl-1,3- und -1,4-xylylendiisocyanat (m- und p-TMXDI), Bis-(1-Isocyanato-1-methylethyl)-naphthalin, $\alpha,\alpha,\alpha',\alpha',\alpha'',\alpha''$-Hexamethyl-1,3,5-mesitylentriisocyanat, Dimer- und Trimerfettsäureisocyanate wie 3,6-Bis-(9-isocyanatononyl)-4,5-di-(1-heptenyl)-cyclohexen (Dimeryldiisocyanat), 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-düsocyanatobenzol, Naphthalin-1,5-diisocyanat (NDI), 3,3'-Dimethyl-4,4'-diisocyanatodiphenyl (TODI), Tris-(4-isocyanatophenyl)-methan, Tris-(4-isocyanatophenyl)-thiophosphat; Oligomere dieser Isocyanate enthaltend Uretdion-, Isocyanurat- oder Iminooxadiazindiongruppen; modifizierte zwei- und mehrwertige Isocyanate enthaltend Ester-, Harnstoff-, Urethan-, Biuret-, Allophanat-, Carbodiimid-, Uretonimin- oder Oxadiazintriongruppen; sowie Isocyanat-haltige Polyurethanpolymere, das heisst mehr als eine Isocyanatgruppe aufweisende Umsetzungsprodukte von Polyisocyanaten mit zwei-oder mehr Hydroxylgruppen aufweisenden Substanzen (so genannten "Polyolen"), wie beispielsweise zwei- oder mehrwertigen Alkoholen, Glykolen oder Aminoalkoholen, polyhydroxyfunktionellen Polyethern, Polyestern, Polyacrylaten, Polycarbonaten oder Polykohlenwasserstoffen, insbesondere Polyethern;
- zwei- oder mehrwertige Epoxide (Polyepoxide) wie Bis-(2,3-epoxycyclopentyl)ether, Polyglycidylether von mehrwertigen aliphatischen und cycloaliphatischen Alkoholen wie 1,4-Butandiol, Polypropylenglykolen und 2,2-Bis-(4-hydroxycyclohexyl)-propan; Polyglycidylether von mehrwertigen Phenolen wie Resorcinol, Bis-(4-hydroxyphenyl)-methan (Bisphenol-F), 2,2-Bis-(4-hydroxyphenyl)-propan (Bisphenol-A), 2,2-Bis-(4-hydroxy-3,5-dibromophenyl)-propan, 1,1,2,2-Tetrakis-(4-hydroxyphenyl)-ethan, Kondensationsprodukten von Phenolen mit Formaldehyd, die unter sauren Bedingungen erhalten werden, wie Phenolnovolaken und Kresolnovolaken, sowie mit diesen Alkoholen und Phenolen, oder mit Polycarbonsäuren wie beispielsweise dimeren Fettsäuren, oder einem Gemisch davon, vorverlängerte Polyglycidylether; Polyglycidylester von mehrwertigen Carbonsäuren wie Phthalsäure, Terephthalsäure, Tetrahydrophthalsäure und Hexahydrophthalsäure; N-Glycidyl-Derivate von Aminen, Amiden und heterocyclischen Stickstoffbasen wie N,N-Diglycidylanilin, N,N-Diglycidyltoluidin, N,N,O-Triglycidyl-4-aminophenol, N,N,N',N'-Tetraglycidyl-bis-(4-aminophenyl)-methan, Triglycidylcyanurat und Triglycidylisocyanurat;
- zwei- oder mehrwertige, Acryl-, Methacryl- oder Acrylamidgruppen tragende Verbindungen wie Tris-(2-hydroxyethyl)-isocyanurat-tri(meth)acrylat, Tris-(2-hydroxyethyl)-cyanurat-tri(meth)acrylat, N,N',N"-Tris-(meth)acryloyl-perhydrotriazin; zwei- oder mehrwertige Acrylate und Methacrylate von aliphatischen Polyethern, Polyestern, Novolaken, Phenolen, aliphatischen oder cycloaliphatischen Alkoholen, Glykolen und Polyesterglykolen sowie mono- und polyalkoxylierten Derivaten der vorgenannten Verbindungen, beispielsweise Ethylenglykol-di(meth)acrylat, Tetraethylenglykol-di(meth)acrylat, Tripropylenglykol-di(meth)acrylat, Polyethylenglykol-di(meth)acrylat, Polypropylenglykoldi(meth)acrylat, 1,4-Butandiol-di(meth)acrylat, 1,6-Hexandiol-di(meth)acrylat, Neopentylglykol-di(meth)acrylat, Trimethylolpropan-tri(meth)acrylat, Pentaerithritol-tetra(meth)acrylat, Dipentaerithritol-tetra(meth)acrylat, Dipentaerithritolpenta(meth)acrylat, Dipentaerithritol-hexa(meth)acrylat; zwei- oder mehrwertige Acryl- oder Methacryl-funktionelle Polybutadiene, Polyisoprene oder Block-copolymere davon; Addukte aus zwei- oder mehrwertigen Epoxiden wie die oben genannten Epoxide mit Acryl- und Methacrylsäure; zwei- oder mehrwertige Polyurethan(meth)acrylate; zwei- oder mehrwertige Acrylamide wie N,N'-Methylen-bis-acrylamid;

- zwei- oder mehrwertige, 1-Ethinylcarbonyl- oder 1-Propinylcarbonylgruppen tragende Verbindungen;
- zwei- oder mehrwertige, Maleimid- oder Citraconimidgruppen tragende Verbindungen wie die Bis- und Polykismaleimide aus aliphatischen, cycloaliphatischen oder aromatischen Di- und Polyaminen und Malein- oder Citraconsäureanhydrid, beispielsweise $\alpha,\omega$-Dimerfettsäure-bis(maleimid), 4,4'-Diphenylmethan-bis(maleimid), 1,3-Xylylen-bis(citraconimid); Bis- und Polykismaleimide aus aminoterminierten Butadien/Acrylonitril-Copolymeren (beispielsweise erhältlich unter dem Namen Hycar® ATBN von Noveon) und Malein-oder Citraconsäureanhydrid; zwei- oder mehrwertige Addukte aus Di- und Polyisocyanaten mit N-Hydroxyethylmaleimid; Ester aus zwei- oder mehrwertigen Alkoholen und 6-Maleimidohexansäure;
- zwei- oder mehrwertige, Vinyl- und/oder Isopropenylgruppen tragende Verbindungen wie 1,3- und 1,4-Divinylbenzol, Divinylsulfon,Vinylcrotonat,Diallylidenpentaerythritol-acetal, 1,3-DÜsopropenylbenzol und 1,3,5-Triisopropenylbenzol, 3-(2-Vinyloxyethoxy)-styrol, Divinyldimethylsilan, Trivinylmethylsilan, Trivinylmethoxysilan, Divinyltetramethyldisiloxan, 1,3-Divinyl-1,3-diphenyl-1,3-dimethyldisiloxan, 1,3-Divinyl-tetraethoxydisiloxan, Trivinyl-pentamethyltrisiloxan, 4-Vinyloxybutoxy-trivinylsilan, Tris-(4-vinyloxybutoxy)-vinylsilan; zwei- oder mehrwertige Vinyl- und Isopropenylether wie Divinylether, Isopropenylvinylether, Triethylenglykol-divinylether, Butandiol-divinylether, Hexandiol-divinylether, Octadecandiol-divinylether, Dimerfettsäurediol-divinylether und Divinylbutyral; Divinylester von Dicarbonsäuren, beispielsweise Adipinsäuredivinylester;
- zwei- oder mehrwertige, Allylgruppen tragende Verbindungen wie Triallylcyanurat, Triallylisocyanurat, Triallylphosphat; zwei- oder mehrwertige Allylether von Alkoholen und Glykolen sowie mono- und polyalkoxylierten Derivaten davon, beispielsweise 1,4-Bis-(allyloxy)-butan, 1,6-Bis-(allyloxy)-hexan, Triethylenglykol-diallylether, Bisphenol-A-diallylether, 3,3'-Diallyl-bisphenol-A-diallylether, 3,3'-Diallyl-bisphenol-A, Trimethylolpropan-diallylether, Glycerintriallylether, Trimethylolpropan-triallylether, Pentaerithritol-tetraallylether; zwei- oder mehrwertige Allylester und -amide von Carbonsäuren, beispielsweise Diallylphthalat, Diallyliso- und -terephthalat, Diallyloxalat, Diallylsebacat, Diallylmaleinat, Diallylfumarat, Diallylitaconat; zweiwertige Allylcarbonate wie Diallylcarbonat, Di- und Triethylenglykol-bis-allylcarbonat; zwei- oder mehrwertige Addukte aus Di- und Polyisocyanaten mit Glycidol, Allylalkohol oder Allylglykolen, beispielsweise 1,6-Hexamethylen-bis-allylcarbamat;
- sowie zwei- oder mehrwertige heterofunktionelle, das heisst mindestens zwei verschiedene der vorgenannten Reaktivgruppen tragende, Verbindungen wie 4-Allyloxy-phenylisocyanat, 1-Alkenylisocyanate wie Vinylisocyanat, Propenylisocyanat und Isopropenylisocyanat, 2-Isocyanatoethyl-methacrylat, 1,2-Dimethyl-3-isocyanatopropyl-acrylat, p-Isocyanatostyrol, m- und p-Isopropenyl-$\alpha,\alpha$-dimethylbenzylisocyanat (m- und p-TMI), m- und p-Ethenyl-$\alpha,\alpha$-dimethylbenzylisocyanat, Isopropenyl-$\alpha,\alpha,\alpha',\alpha'$-tetramethyl-xylylendiisocyanat, Glycidoxy-trivinylsilan, Triglycidoxy-vinylsilan, N-(Trivinylsilyloxymethyl)-maleimid; heterofunktionelle Addukte aus Di- und Polyisocyanaten mit Glycidol, Allylalkohol, Allylglykolen, N-Hydroxyethylmaleimid, hydroxyfunktionellen Acrylat- und Methacrylaten wie 2-Hydroxyethyl-acrylat und -methacrylat; heterofunktionelle Addukte aus Mono- und Polycarbodümiden von Di- und Polyisocyanaten mit Acryl- oder Methacrylsäure; heterofunktionelle Addukte aus zwei-oder mehrwertigen Epoxiden mit Acryl- oder Methacrylsäure, Vinylallylether, Ethylenglykol-vinylallylether, Vinylallylphthalat, Ethylenglykol-(2-allylphenyl)-vinyl-ether, Allyl(meth)acrylat, Vinylacrylat, 2-Vinyloxyethyl-(meth)acrylat.

Als Verbindungen **D** insbesondere geeignet sind zwei- oder mehrwertige aliphatische, cycloaliphatische, arylaliphatische und aromatische Isocyanate wie die erwähnten monomeren und oligomeren Polyisocyanate sowie die mehr als eine Isocyanatgruppe aufweisenden Umsetzungsprodukte von Polyisocyanaten mit Polyolen, insbesondere Polyetherpolyolen, Polyesterpolyolen, Polyacrylatpolyolen, Polycarbonatpolyolen, Polykohlenwasserstoffpolyolen und Mischungen dieser Polyole.

Je nach den Reaktivgruppen der Verbindung **D** und der den aktiven Wasserstoff tragenden Gruppe des Aldimins der Formel (I) kann die zur Aldimin-haltigen Verbindung **AV** führende Additionsreaktion nucleophil oder radikalisch erfolgen. Aus Gründen der Einfachheit soll der Begriff "Additionsreaktion" im vorliegenden Dokument auch ringöffnende Substitutionsreaktionen, wie sie beispielsweise Epoxide mit Nucleophilen eingehen, umfassen, weil das Ergebnis einer solchen, das Nucleofug nicht als separates Molekül freisetzenden, Substitutionsreaktion dem einer Additionsreaktion gleichkommt. Die Additionsreaktion verläuft nucleophil, wenn die den aktiven Wasserstoff tragende Reaktivgruppe des Aldimins als Nucleophil an eine elektrophile Reaktivgruppe der Verbindung **D** angreift, beispielsweise im Fall des Angriffs einer Amino- oder Hydroxylgruppe an eine Isocyanatgruppe. Als Beispiel für eine radikalische Additionsreaktion kann die Reaktion einer Mercaptogruppe an eine Acrylgruppe genannt werden, wobei für diese Art der Additionsreaktion im Allgemeinen ein radikalbildender Initiator benötigt wird.

Die Umsetzung zwischen dem Aldimin der Formel (I) und der Verbindung **D** zur Aldimin-haltigen Verbindung **AV** erfolgt unter bekannten Bedingungen, wie sie für Reaktionen zwischen den an der jeweiligen Umsetzung beteiligten Reaktivgruppen typischerweise verwendet werden, beispielsweise bei 20 bis 100°C. Die Umsetzung erfolgt unter Verwendung eines Lösemittels oder bevorzugt lösemittelfrei. Gegebenenfalls können Hilfsstoffe wie beispielsweise Katalysatoren, Initiatoren oder Stabilisatoren mitverwendet werden. Die Umsetzung mit Isocyanaten wird für Aminoaldimine

bevorzugt bei Raumtemperatur und ohne Katalysator, für Hydroxy-, Mercapto-und Harnstoffaldimine bei 40 bis 100°C und unter Verwendung eines Katalysators, wie er für die Urethanisierungsreaktion zwischen Isocyanaten und Alkoholen verwendet wird, beispielsweise einer Organozinn-Verbindung, eines Bismutkomplexes, einer tertiären Aminverbindung oder einer Kombination solcher Katalysatoren, durchgeführt.

Die auf die beschriebene Weise erhaltenen Aldimin-haltigen Verbindungen **AV** sind, wie die Aldimine der Formel (I), geruchsfrei. Sie sind unter geeigneten Bedingungen, insbesondere unter Ausschluss von Feuchtigkeit, lagerstabil. Heterofunktionelle Aldimin-haltigen Verbindungen **AV**, die neben Aldiminogruppen zusätzliche, für Polyreaktionen zugängliche Reaktivgruppen enthalten, sind dann lagerstabil, wenn sie überdies von Reaktionen dieser Reaktivgruppen auslösenden Faktoren, wie beispielsweise Hitze oder UV-Strahlung, ferngehalten werden.

Bei Zutritt von Feuchtigkeit können die Aldiminogruppen der Aldimin-haltigen Verbindungen **AV** über Zwischenstufen formal zu Aminogruppen hydrolysieren, wobei der entsprechende, zur Herstellung des Aldimins verwendete Aldehyd **A** freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen teilweise oder vollständig hydrolysieren. Im speziellen Fall von heterofunktionellen Aldimin-haltigen Verbindungen **AV,** die gegenüber Aminen reaktive Gruppen, insbesondere Isocyanatgruppen, enthalten, reagieren die hydrolysierenden Aldiminogruppen hingegen weiter, beispielsweise mit Isocyanatgruppen zu Harnstoffgruppen. In diesem Fall kommt es zur Vernetzung der heterofunktionellen Aldimin-haltigen Verbindung **AV,** welche auch ohne Beteiligung weiterer Substanzen direkt zu einem hochmolekularen Kunststoff führen kann. Die Reaktion der gegenüber Aminen reaktiven Gruppen mit den hydrolysierenden Aldiminogruppen muss dabei nicht notwendigerweise über Aminogruppen erfolgen. Selbstverständlich sind auch Reaktionen mit Zwischenstufen der Hydrolysereaktion möglich. Beispielsweise ist es denkbar, dass die hydrolysierende Aldiminogruppe in der Form eines Halbaminals direkt mit den gegenüber Aminen reaktiven Gruppen reagiert.

Als Katalysatoren für die Hydrolyse der Aldiminogruppen eignen sich beispielsweise organische Carbonsäuren wie Benzoesäure, Salicylsäure oder 2-Nitrobenzoesäure, organische Carbonsäureanhydride wie Phthalsäureanhydrid oder Hexahydrophthalsäureanhydrid, Silylester von organischen Carbonsäuren, organische Sulfonsäuren wie Methansulfonsäure, p-Toluolsulfonsäure oder 4-Dodecylbenzolsulfonsäure, oder andere organische oder anorganische Säuren, oder Mischungen der vorgenannten Säuren.

Wie bereits erwähnt können die vorgängig beschriebenen Aldimin-haltigen Verbindungen **AV** zur Herstellung von Kunststoffvorläufern verwendet werden. Geeignete Kunststoffvorläufer, in welchen sich die Aldimin-haltigen Verbindungen **AV** als Bausteine, beispielsweise als latente Härter oder als Co-Monomere, einsetzen lassen, sind solche, welche Substanzen mit mindestens einem Typ von Reaktivgruppen enthalten, die mit Aldiminen als solchen oder nach deren teilweisen oder vollständigen Hydrolyse Reaktionen eingehen, die für sich allein oder in Kombination mit weiteren Reaktionen zur Vernetzung des Kunststoffvorläufers führen. Beispiele für solche Reaktivgruppen sind Isocyanat-, Isothiocyanat-, Epoxid-, Episulfid- und Cyclocarbonatgruppen. Die Reaktionen dieser Reaktivgruppen mit den Aldiminogruppen können durch Feuchtigkeit und/oder durch Hitze ausgelöst werden. Insbesondere geeignete Reaktivgruppen sind Isocyanatgruppen, Isothiocyanatgruppen und Epoxidgruppen. Als Kunststoffvorläufer geeignet sind auch solche Kunststoffvorläufer, welche neben den Substanzen mit den genannten Reaktivgruppen weitere Substanzen mit für Polyreaktionen zugänglichen Gruppen, wie beispielsweise Aziridin-, Acryl-, Methacryl-, 1-Ethinylcarbonyl-, 1-Propinylcarbonyl-, Maleimid-, Citraconimid-, Vinyl-, Isopropenyl-, Allyl- oder Silanolgruppen, oder zu Silanolgruppen hydrolysierende Gruppen, enthalten.

Insbesondere geeignete Kunststoffvorläufer, in welchen sich die Aldimin-haltigen Verbindungen **AV** einsetzen lassen, sind Isocyanat-haltige Zusammensetzungen, das heisst solche Kunststoffvorläufer, welche als Reaktivgruppen ausschliesslich oder zu einem wesentlichen Teil Isocyanatgruppen enthalten, die Bestandteil von Polyurethanpolymeren und/oder von Polyisocyanaten sind.

Geeignete Kunststoffvorläufer, welche die Aldimin-haltigen Verbindungen **AV** als Bestandteile enthalten, können einkomponentig oder zweikomponentig sein.

Als einkomponentige Kunststoffvorläufer insbesondere geeignet sind einkomponentige Isocyanat-haltige Zusammensetzungen, welche mindestens eine Aldimin-haltige Verbindung **AV** und mindestens ein Isocyanat-haltiges Polyurethanpolymer **P,** das ein Umsetzungsprodukt aus Polyisocyanaten und Polyolen darstellt, enthalten. Der Begriff "Polyurethanpolymer" umfasst im vorliegenden Dokument sämtliche Polymere, welche nach dem Diisocyanat-Polyadditions-Verfahren hergestellt werden. Dies schliesst auch solche Polymere ein, die nahezu oder gänzlich frei sind von Urethangruppen, wie Polyether-Polyurethane, Polyester-Polyurethane, Polyether-Polyharnstoffe, Polyharnstoffe, Polyester-Polyharnstoffe, Polyisocyanurate, Polycarbodiimide, usw.

Das Isocyanat-haltige Polyurethanpolymer **P** wird hergestellt durch Umsetzung von mindestens einem Polyol mit mindestens einem Polyisocyanat. Diese Umsetzung kann dadurch erfolgen, dass das Polyol und das Polyisocyanat mit üblichen Verfahren, beispielsweise bei Temperaturen von 50°C bis 100° C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind. Der

Überschuss an Polyisocyanat wird so gewählt, dass im resultierenden Polyurethanpolymer P nach der Umsetzung aller Hydroxylgruppen des Polyols beispielsweise ein Gehalt an freien Isocyanatgruppen von 0.1 - 15 Gewichts-%, insbesondere 0.5 - 5 Gewichts-%, bezogen auf das gesamte Polyurethanpolymer **P,** verbleibt. Gegebenenfalls kann das Polyurethanpolymer **P** unter Mitverwendung von Weichmachern hergestellt werden, wobei die verwendeten Weichmacher keine gegenüber Isocyanaten reaktiven Gruppen enthalten.
Als Polyole für die Herstellung eines solchen Isocyanat-haltigen Polyurethanpolymers **P** können beispielsweise die folgenden, handelsüblichen Polyole, oder beliebige Mischungen davon, eingesetzt werden:

- Polyoxyalkylenpolyole, auch Polyetherpolyole oder Oligoetherole genannt, welche Polymerisationsprodukte von Ethylenoxid, 1,2-Propylenoxid, 1,2- oder 2,3-Butylenoxid, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.
  Besonders geeignet sind Polyoxyalkylendiole oder Polyoxyalkylentriole, insbesondere Polyoxypropylendiole oder Polyoxypropylentriole. Speziell geeignet sind Polyoxyalkylendiole oder Polyoxyalkylentriole mit einem Ungesättigtheitsgrad tiefer als 0.02 mEq/g und mit einem Molekulargewicht im Bereich von 1 ’000 - 30’000 g/mol, sowie Polyoxypropylendiole und -triole mit einem Molekulargewicht von 400 - 8’000 g/mol. Der Begriff "Molekulargewicht" bezeichnet im vorliegenden Dokument das Molekulargewichtsmittel $M_{n.}$
  Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole mit organischen Dicarbonsäuren oder deren Anhydride oder Ester wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Polyacrylat- und Polymethacrylatpolyole.
- Polykohlenwasserstoffpolyole, auch Oligohydrocarbonole genannt, wie beispielsweise polyhydroxyfunktionelle Ethylen-Propylen-, Ethylen-Butylen-oder Ethylen-Propylen-Dien-Copolymere, wie sie beispielsweise von der Firma Kraton Polymers hergestellt werden, oder polyhydroxyfunktionelle Copolymere aus Dienen wie 1,3-Butandien oder Diengemischen und Vinylmonomeren wie Styrol, Acrylnitril oder Isobutylen, oder polyhydroxyfunktionelle Polybutadienpolyole, wie beispielsweise solche, die durch Copolymerisation von 1,3-Butadien und Allylalkohol hergestellt werden.
- Polyhydroxyfunktionelle Acrylonitril/Polybutadien-Copolymere, wie sie beispielsweise aus Epoxiden oder Aminoalkoholen und carboxylterminierten Acrylonitril/Polybutadien-Copolymeren (kommerziell erhältlich unter dem Namen Hycar® CTBN von Hanse Chemie) hergestellt werden können.

Diese genannten Polyole weisen ein mittleres Molekulargewicht von 250 - 30’000 g/mol, insbesondere von 1 ’000 - 30’000 g/mol, und eine mittlere OH-Funktionalität im Bereich von 1.6 bis 3 auf.
Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere

Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **P** mitverwendet werden.

Als Polyisocyanate für die Herstellung eines solchen Isocyanat-haltigen Polyurethanpolymers werden die mono- oder oligomeren zwei- oder mehrwertigen Isocyanate verwendet, wie sie als Verbindungen D geeignet genannt wurden. Als Polyisocyanate insbesondere geeignet sind MDI, HDI, TDI und IPDI.

Der einkomponentige Kunststoffvorläufer enthält mindestens eine Aldimin-haltige Vewrbindung **AV**, insbesondere in einer der oben bereits im Detail beschriebenen bevorzugten Ausführungsform. Die Aldimin-haltige Verbindung **AV** kann dabei gesondert hergestellt und als solche in den Kunststoffvorläufer eingearbeitet werden. Sie kann aber auch in situ, das heisst im Zuge der Herstellung des Kunststoffvorläufers, hergestellt werden, indem geeignete Mengen von mindestens einem Aldimin der Formel (I) und mindestens einer Verbindung **D** in situ, das heisst in Anwesenheit weiterer Bestandteile des Kunststoffvorläufers, zur Aldimin-haltigen Verbindung **AV** umgesetzt werden. Insbesondere kann zur Herstellung der beschriebenen einkomponentigen Isocyanat-haltigen Zusammensetzungen so vorgegangen werden, dass geeignete Mengen von mindestens einem Aldimin der Formel (I) und mindestens einer Verbindung **D** in situ umgesetzt werden, wobei die Verbindung **D** bevorzugt ein Isocyanat-haltiges Polyurethanpolymer darstellt, wie es oben als Polyurethanpolymer **P** im Detail beschrieben wurde.

Typischerweise ist die Aldimin-haltige Verbindung **AV** in einer Menge von 0.1 bis 30 Gewichts-%, bevorzugt 0.5 bis 20 Gewichts-%, und insbesondere 1 bis 10 Gewichts-%, bezogen auf den einkomponentigen Kunststoffvorläufer, insbesondere die einkomponentige Isocyanat-haltige Zusammensetzung, vorhanden.

Für den Fall, dass die Aldimin-haltige Verbindung **AV** ein freie Isocyanatgruppen aufweisendes Umsetzungsprodukt aus einem Isocyanat-haltigen Polyurethanpolymer und einem Aldimin der Formel (I) darstellt, kann der Gehalt der Aldimin-haltigen Verbindung **AV** im einkomponentigen Kunststoffvorläufer auch bis gegen 100 Gewichts-% betragen, da eine derartige Zusammensetzung unter dem Einfluss von Feuchtigkeit vernetzt.

Es ist vorteilhaft,wenn die einkomponentige Isocyanat-haltige Zusammensetzung zusätzlich zur Aldimin-haltigen Verbindung **AV** und zum Polyurethanpolymer **P** mindestens einen Katalysator **KAT-1** enthält. Als Katalysator **KAT-1** geeignet sind Verbindungen, welche zusammen mit Isocyanatgruppen lagerstabil sind, und welche die zur Aushärtung der Zusammensetzung führenden Reaktionen der Isocyanatgruppen, insbesondere jene mit Aldiminogruppen sowie mit Feuchtigkeit, beschleunigen. Als geeignete Katalysatoren **KAT-1** genannt werden sollen Säuren, beispielsweise organische Carbonsäuren wie Benzoesäure, Salicylsäure oder 2-Nitrobenzoesäure, organische Carbonsäureanhydride wie Phthalsäureanhydrid oder Hexahydrophthalsäureanhydrid, Silylester von organischen Carbonsäuren, organische Sulfonsäuren wie Methansulfonsäure, p-Toluolsulfonsäure oder 4-Dodecylbenzolsulfonsäure, oder weitere organische oder anorganische Säuren; Metallverbindungen, beispielsweise Zinnverbindungen, zum Beispiel Dialkylzinn(II)-dicarboxylate wie Dibutylzinndiacetat, Dibutylzinn-bis-(2-ethyl-hexanoat), Dibutylzinndilaurat, Dibutylzinndipalmitat, Dibutylzinndistearat, Dibutylzinndioleat, Dibutylzinndilinolat, Dibutylzinndilinolenat, Dibutylzinndiacetylacetonat, Dibutylzinnmaleat, Dibutylzinn-bis-(octylmaleinat), Dibutylzinnphthalat, Dimethylzinndilaurat, Dioctylzinndiacetat oder Dioctylzinndilaurat, Dialkylzinnmercaptide wie Dibutylzinn-bis-(2-ethylhexyl mercaptoacetat) oder Dioctylzinn-bis-(2-ethylhexyl mercaptoacetat), Dibutylzinndichlorid, Monobutylzinntrichlorid, Alkylzinnthioester, Dibutylzinnoxid, Dioctylzinnoxid, Zinn(II)-carboxylate wie Zinn(II)-octoat, Zinn(II)-2-ethylhexanoat, Zinn(II)-laurat, Zinn(II)-oleat oder Zinn(II)-naphthenat, Stannoxane wie Laurylstannoxan, Bismutverbindungen wie Bismut(III)-octoat, Bismut(III)-neodecanoat oder Bismut(III)-oxinate; schwach basische tertiäre Aminverbindungen wie beispielsweise 2,2'-Dimorpholinodiethylether und andere Morpholinether-Derivate; sowie Kombinationen der genannten Verbindungen, insbesondere von Säuren und Metallverbindungen oder von Metallverbindungen und aminogruppenhaltigen Verbindungen.

Der einkomponentige Kunststoffvorläufer enthält gegebenenfalls weitere Bestandteile, wie sie nach dem Stand der Technik üblicherweise eingesetzt werden. Insbesondere enthält die einkomponentige Isocyanat-haltige Zusammensetzung gegebenenfalls eines oder mehrere der folgenden Hilfs- und Zusatzmittel:

- Weichmacher, beispielsweise Ester organischer Carbonsäuren oder deren Anhydride, Phthalate, wie beispielsweise Dioctylphthalat oder Diisodecylphthalat, Adipate, wie zum Beispiel Dioctyladipat, Sebacate, Polyole wie beispielsweise Polyoxyalkylenpolyole oder Polyesterpolyole, organische Phosphor- und Sulfonsäureester oder Polybutene;
- Lösemittel, beispielsweise Ketone wie Aceton, Methylethylketon, Diisobutylketon,Acetonylaceton,Mesityloxid,sowie cyclische Ketone wie Methylcyclohexanon und Cyclohexanon; Ester wie Ethylacetat, Propylacetat oder Butylacetat, Formiate, Propionate oder Malonate; Ether wie Ketonether, Esterether und Dialkylether wie Diisopropylether, Diethylether, Dibutylether, Diethylenglykoldiethylether sowie Ethylenglykoldiethylether; aliphatische und aromatische Kohlenwasserstoffe wie Toluol, Xylol, Heptan, Octan sowie unterschiedliche Erdölfraktionen wie Naphtha, White Spirit, Petrolether oder Benzin;halogenierte Kohlenwasserstoffe wie Methylenchlorid; sowie N-alkylierte Lactame wie beispielsweise N-Methylpyrrolidon,N-Cyclohexylpyrrolidon oder N-Dodecylpyrrolidon;
- anorganische und organische Füllstoffe, wie zum Beispiel gemahlene oder gefällte Calciumcarbonate, welche gegebenenfalls mit Stearaten beschichtet sind, insbesondere feinteiliges beschichtetes Calciumcarbonat, Russe, Kaoline, Aluminiumoxide, Kieselsäuren, PVC-Pulver oder Hohlkugeln; Fasern, beispielsweise aus Polyethylen; Pigmente;

- weitere in der Polyurethanchemie übliche Katalysatoren;
- Reaktivverdünner und Vernetzer, beispielsweise Polyisocyanate wie MDI, PMDI, TDI, HDI, 1,12-Dodecamethylendüsocyanat, Cyclohexan-1,3- oder 1,4-diisocyanat, IPDI, Perhydro-2,4'- und -4,4'-diphenylmethandüsocyanat, 1,3- und 1,4-Tetramethylxylylendiisocyanat, Oligomere und Polymere dieser Polyisocyanate, insbesondere Isocyanurate, Carbodiimide, Uretonimine, Biurete, Allophanate und Iminooxadiazindione der genannten Polyisocyanate, Addukte von Polyisocyanaten mit kurzkettigen Polyolen, sowie Adipinsäuredihydrazid und andere Dihydrazide;
- latente Polyamine wie beispielsweise Polyaldimine, Polyketimine, Polyenamine, Polyoxazolidine, an einen Zeolith adsorbierte oder mikroverkapselte Polyamine sowie Amin-Metallkomplexe, bevorzugt Polyaldimine aus der Umsetzung eines primären aliphatischen Polyamins mit einem Aldehyd, insbesondere einem Aldehyd **A** wie beispielsweise 2,2-Dimethyl-3-acyloxy-propanal, insbesondere 2,2-Dimethyl-3-lauroyloxy-propanal, sowie Komplexe zwischen Methylendianilin (MDA) und Natriumchlorid (erhältlich als Dispersion in Diethylhexylphthalat oder Diisodecylphthalat unter dem Handelsnamen Caytur® 21 von Crompton Chemical);
- Trocknungsmittel, wie beispielsweise p-Tosylisocyanat und andere reaktive Isocyanate, Orthoameisensäureester, Calciumoxid; Vinyltrimethoxysilan oder andere schnell hydrolysierende Silane wie beispielsweise Organoalkoxysilane, welche in $\alpha$-Stellung zur Silangruppe eine funktionelle Gruppe aufweisen, oder Molekularsiebe;
- Rheologie-Modifizierer wie beispielsweise Verdickungsmittel, zum Beispiel Harnstoffverbindungen, Polyamidwachse, Bentonite oder pyrogene Kieselsäuren;
- Haftvermittler, insbesondere Silane wie beispielsweise Epoxysilane,Vinylsilane, (Meth)acrylsilane, Isocyanatosilane, Carbamatosilane, S-(Alkylcarbonyl)-mercaptosilane und Aldiminosilane, sowie oligomere Formen dieser Silane;
- Stabilisatoren gegen Wärme, Licht- und UV-Strahlung; flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen;

sowie weitere üblicherweise in einkomponentigen Isocyanat-haltigen Zusammensetzungen eingesetzte Substanzen.

Der einkomponentige Kunststoffvorläufer, insbesondere die einkomponentige Isocyanat-haltige Zusammensetzung, verfügt in Abwesenheit von die Vernetzungsreaktionen der im Kunststoffvorläufer vorhandenen Reaktivgruppen auslösenden Faktoren, insbesondere von Feuchtigkeit, Hitze oder UV-Strahlung, über eine gute Lagerstabilität. Insbesondere verfügt die einkomponentige Isocyanat-haltige Zusammensetzung in Abwesenheit von Feuchtigkeit, beispielsweise in einer klimadichten Verpackung oder Anordnung, wie beispielsweise in einem Fass, einem Beutel oder einer Kartusche, über eine gute Lagerstabilität. Mit den Begriffen "lagerstabil" und "Lagerstabilität" in Zusammenhang mit einem Kunststoffvorläufer wird im vorliegenden Dokument der Sachverhalt bezeichnet, dass die Viskosität des Kunststoffvorläufers bei geeigneter Lagerung in der betrachteten Zeitspanne nicht oder höchstens so stark ansteigt, dass der Kunststoffvorläufer auf die vorgesehene Weise verwendbar bleibt.

Unter dem Einfluss von Feuchtigkeit, beispielsweise beim Kontakt mit feuchter Luft oder nach dem Zumischen von Wasser, oder bei starker Erwärmung, oder unter dem Einfluss von UV-Strahlung, oder unter dem Einfluss einer Kombination dieser Faktoren, härtet der Kunststoffvorläufer rasch zu einem hochmolekularen Kunststoff aus. Insbesondere härtet die Isocyanat-haltige Zusammensetzung unter dem Einfluss von Feuchtigkeit schnell und vollständig zu einem weitgehend klebefreien Polyurethankunststoff aus. Die Aushärtung verläuft ohne Blasenbildung, da die Isocyanatgruppen teilweise oder vollständig mit den hydrolysierenden Aldiminogruppen reagieren, wobei nur wenig oder gar kein $CO_2$ entsteht. Die Aushärtung wird durch die Anwesenheit von Katalysatoren für die Hydrolyse der Aldiminogruppen, beispielsweise die bereits genannten organischen Carbonsäuren oder Sulfonsäuren, zusätzlich beschleunigt, ohne dass es dabei zur Blasenbildung kommt. Die zur Aushärtung benötigte Feuchtigkeit kann aus der Luft stammen (Luftfeuchtigkeit), wobei der Kunststoffvorläufer durch die Diffusion der Feuchtigkeit von aussen nach innen aushärtet. Der Kunststoffvorläufer kann aber auch mit einer Wasser enthaltenden Komponente in Kontakt gebracht werden, zum Beispiel durch Bestreichen, beispielsweise mit einem Abglättmittel, durch Besprühen oder mittels Eintauchverfahren, oder es kann dem Kunststoffvorläufer eine Wasser enthaltende Komponente zugesetzt werden, zum Beispiel in Form einer wasserhaltigen Paste, die beispielsweise über einen Statikmischer homogen oder heterogen mit dem Kunststoffvorläufer vermischt wird.

Wie bereits erwähnt, können geeignete Kunststoffvorläufer, welche die beschriebenen Aldimin-haltigen Verbindungen **AV** als Bestandteile enthalten, auch zweikomponentig sein. Geeignete zweikomponentige Kunststoffvorläufer bestehen aus zwei Komponenten **K1** und **K2,** wobei mindestens eine der Komponenten **K1** oder **K2** mindestens eine Aldimin-haltige Verbindungen **AV** enthält, und wobei die Mischung der beiden Komponenten **K1** und **K2** zu einem hochmolekularen Kunststoff führt.

Als zweikomponentige Kunststoffvorläufer insbesondere geeignet sind zweikomponentige Isocyanat-haltige Zusammensetzungen, in welchen die Komponente **K1** mindestens ein Polyisocyanat und/oder mindestens ein Isocyanat-haltiges Polyurethanpolymer **P** umfasst, und die Komponente **K2** mindestens ein Polyol und/oder mindestens ein Polyamin umfasst, und mindestens eine der Komponenten **K1** oder **K2** mindestens eine Aldimin-haltige Verbindung **AV** enthält.

Als Polyisocyanat der Komponente **K1** geeignet sind die für die Herstellung des Isocyanat-haltigen Polyurethanpolymers **P** genannten Polyisocyanate. Insbesondere geeignet ist PMDI ("polymeres MDI"), bekannt beispielsweise unter Handelsnamen wie Desmodur® VL, Desmodur® VL 50, Desmodur® VL R 10, Desmodur® VL R 20, Desmodur® VKS 20 F (alle von Bayer), Isonate® M 309, Voranate® M 229, Voranate M® 580 (alle von Dow) oder Lupranat® M 10 R (von BASF), sowie bei Raumtemperatur flüssige Formen von MDI (sogenanntes "modifiziertes MDI"), welche Gemische von MDI mit MDI-Derivaten, wie beispielsweise MDI-Carbodiimiden oder MDI-Uretoniminen, darstellen, bekannt beispielsweise unter Handelsnamen wie Desmodur® CD, Desmodur® PF, Desmodur® PC (alle von Bayer).

Als Isocyanat-haltige Polyurethan polymere **P** der Komponente **K1** insbesondere geeignet sind solche, welche unter Verwendung von MDI, HDI, TDI oder IPDI hergestellt wurden.

Als Polyole der Komponente **K2** geeignet sind dieselben Polyole, welche bereits als geeignet für die Herstellung des Isocyanat-haltigen Polyurethanpolymers P genannt wurden. Insbesondere geeignet sind hochfunktionelle Polyole, beispielsweise Triole, Tetrole und höherfunktionelle Polyole; aminhaltige bzw. mit Aminen (beispielsweise Ethylendiamin) gestartete Polyetherpolyole; kurzkettige Polyetherpolyole mit Molekulargewichten von 300 bis 2000; hydrophobe Polyole, insbesondere Fettpolyole wie beispielsweise Ricinusöl oder die unter dem Handelsnamen Sovermol® von Cognis bekannten Polyole; ebenso Diol-Kettenverlängerer wie 1,4-Butandiol, 1,6-Hexandiol, Ethylenglykol, Diethylenglykol, Propylenglykol, Dipropylenglykol, 1,4-Bis-(hydroxyethyl)-hydrochinon, 1,4-Cyclohexandiol oder N,N'-Bis-(hydroxyethyl)-piperazin.

Als Polyamine der Komponente **K2** geeignet sind zum einen primäre aliphatische Polyamine, wie sie als Amine **C** beschrieben wurden; zum anderen Polyaminoamide; sekundäre aliphatische Polyamine wie beispielsweise N,N'-Dibutylethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink® 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, N-alkylierte Polyetheramine, Produkte aus der Michael-artigen Addition der beispielhaft erwähnten primären aliphatischen Polyamine an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd; aliphatische Polyamine mit primären und sekundären Aminogruppen, wie beispielsweise N-Butyl-1,6-hexandiamin; sowie primäre und/oder sekundäre aromatische Polyamine wie beispielsweise m- und p-Phenylendiamin, 4,4'-Diaminodiphenylmethan (MDA), 3,3'-Dichloro-4,4'-diaminodiphenylmethan (MOCA), Mischungen von 3,5-Dimethylthio-2,4- und -2,6-toluylendiamin (erhältlich als Ethacure® 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink® von Air Products), 1,2-Bis-(2-aminophenylthio)-ethan, N,N'-Dialkyl-p-phenylendiamin, N,N'-Dialkyl-4,4'-diaminodiphenylmethan, 2-Methylpropyl-(4-chloro-3,5-diaminobenzoat) und tert.Butyl-(4-chloro-3,5-diaminobenzoat.

Es ist auch möglich, Polyamine in Form von Derivaten einzusetzen, in denen alle oder ein Teil der Aminogruppen blockiert sind und erst nach ihrer Aktivierung durch Hydrolyse und/oder Erwärmen mit Isocyanaten reagieren. Beispiele für solche Polyamin-Derivate mit blockierten Aminogruppen sind mehrfunktionelle Aldimine, Ketimine, Enamine, Oxazolidine, Aminale, Ammoniumcarbonate, Amin-Kohlensäure-Salze (Carbamate) oder Amin-Metallkomplexe. Ebenfalls können an Zeolith adsorbierte oder mikroverkapselte Polyamine eingesetzt werden.

Die zweikomponentige Isocyanat-haltige Zusammensetzung enthält mindestens eine Aldimin-haltige Verbindung **AV,** insbesondere in einer der oben bereits im Detail beschriebenen bevorzugten Ausführungsform.

Typischerweise ist die Aldimin-haltige Verbindung **AV** in einer Menge von 0.1 bis 50 Gewichts-%, bevorzugt 0.5 bis 30 Gewichts-%, und insbesondere 1 bis 20 Gewichts-%, bezogen auf die zweikomponentige Isocyanat-haltige Zusammensetzung, vorhanden.

Es ist vorteilhaft,wenn die zweikomponentige Isocyanat-haltige Zusammensetzung mindestens einen Katalysator **KAT-2** enthält.Als Katalysator **KAT-2** geeignet sind Verbindungen, welche die Aushärtung der Zusammensetzung beschleunigen. Als geeignete Katalysatoren **KAT-2** genannt werden sollen einerseits die bereits genannten Katalysatoren **KAT-1,** sowie weitere Katalysatoren, beispielsweise Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink (II)-acetat, Zink(II)-2-ethylhexanoat, Zink(II)-laurat, Zink(II)-oleat, Zink(II)-naphthenat, Zink(II)-acetylacetonat, Zink(II)-Salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilberneodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kalium-octoat; tertiäre Aminverbindungen wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo [2.2.2]octan, 1,8-Diazabicyclo [5.4.0]undec-7-en (DBU), N-Methylmorpholin, N-Ethylmorpholin, N-Cocomorpholin, N, N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin oder Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Aminverbindungen enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propyl-harnstoff, Mannich-Basen wie 2,4,6-Tris-(dimethylaminomethyl)-phenol oder 2,4,6-Tris-(3-(dimethylamino)-propylaminomethyl)-phenol, N-Hydroxypropylimidazol, N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metall-und Aminogruppenhaltigen Verbindungen.

Zusätzlich zur Aldimin-haltigen Verbindung **AV,** zum Polyisocyanat oder Isocyanat-haltigen Polyurethanpolymer **P,** zum Polyol und/oder Polyamin und zum gegebenenfalls vorhandenen Katalysator **KAT-2** kann die zweikomponentige Isocyanat-haltige Zusammensetzung weitere Bestandteile enthalten, wobei dieselben Weichmacher, Lösemittel, Füllstoffe, Katalysatoren, Reaktivverdünner und Vernetzer, latente Polyamine, Trocknungsmittel, Rheologiemodifizierer, Haftvermittler, Stabilisatoren, oberflächenaktiven Substanzen und Biocide eingesetzt werden können, welche bereits für die einkomponentige Zusammensetzung genannt wurden, sowie weitere üblicherweise in zweikomponentigen Polyurethanzusammensetzungen eingesetzte Substanzen. Die Aufteilung dieser zusätzlichen Bestandteile auf die Komponenten **K1** und **K2** erfolgt in der dem Fachmann für zweikomponentige Polyurethanzusammensetzungen bekannten Weise.

Die Komponenten **K1** und **K2** sind, getrennt voneinander aufbewahrt, jeweils lagerstabil. Insbesondere die Komponente **K1** muss unter Ausschluss von Feuchtigkeit hergestellt und gelagert werden.

Die beiden Komponenten **K1** und **K2** werden erst kurz vor der Applikation auf eine geeignete Weise miteinander vermischt, wobei darauf geachtet werden muss, dass beim Mischvorgang möglichst wenig Luft in die gemischte Zusammensetzung gelangt, und dass ein geeignetes Mischungsverhältnis eingehalten wird.Sobald die beiden Komponenten miteinander in Kontakt kommen, beginnen die in ihnen enthaltenen reaktiven Bestandteile miteinander zu reagieren und führen so zur Aushärtung der gemischten zweikomponentigen Zusammensetzung. Insbesondere reagieren die Isocyanatgruppen der Komponente **K1** mit den partiell oder vollständig hydrolysierten Aldiminogruppen der Komponente **K1** oder **K2** sowie mit den Hydroxyl- und/oder Amingruppen der Komponente **K2.** Die Aushärtung der gemischten zweikomponentigen Zusammensetzung kann bei Raumtemperatur erfolgen; gegebenenfalls kann sie auch durch Zufuhr von Wärme beschleunigt werden, insbesondere dann, wenn die Zusammensetzung langsam reagierende Polyole oder Polyisocyanate enthält, oder wenn sie thermisch latente Polyamine,wie Amin-Metallkomplexe oder mikroverkapselte Polyamine, enthält, welche erst nach Überschreiten einer Aktivierungstemperatur, beispielsweise 80-200°C, mit den Isocyanatgruppen reagieren.

Das Mischungsverhältnis zwischen den Komponenten **K1** und **K2** wird üblicherweise so gewählt, dass ein gewisser Überschuss an Isocyanatgruppen in Bezug auf mit Isocyanatgruppen reagierende Gruppen wie Aldimino-, Hydroxyl- und Aminogruppen vorhanden ist. Üblicherweise wird das Mischungsverhältnis so gewählt, dass das Verhältnis ([OH] + [NH]) / [NCO] einen Wert von 0.5 bis 0.95 aufweist. Dadurch wird sichergestellt, dass die gemischte zweikomponentige Zusammensetzung zu einem polymeren Material aushärtet, wobei überschüssige Isocyanatgruppen entweder mit Feuchtigkeit aus der Komponente **K2** oder mit Feuchtigkeit aus der Luft reagieren. Ebenfalls muss darauf geachtet werden, dass zwischen dem Vermischen der Komponenten **K1** und **K2** und dem Applizieren auf eine Oberfläche eines Substrates nicht zuviel Zeit vergeht, da ein zu starkes Vorreagieren vor der Applikation das Ausbilden einer guten Haftung zum Untergrund erschwert.

In einer weiteren bevorzugten Verwendungsart werden die Aldimine der Formel (I) direkt, das heisst ohne

Umsetzung zu Additionsprodukten, als Bestandteile von Kunststoffvorläufern eingesetzt, beispielsweise als latente Härter oder als Co-Monomere, insbesondere für zweikomponentige Isocyanat-haltige Zusammensetzungen. Es hat sich überraschenderweise gezeigt, dass insbesondere solche Aldimine der Formel (I), die mehr als eine sekundäre Aminogruppe tragen, in Isocyanat-haltigen Zusammensetzungen gleichzeitig die Funktionen eines latenten Härters, eines Trocknungsmittels und eines Thixotropiermittels übernehmen können. Insbesondere geeignete zweikomponentige Isocyanat-haltige Zusammensetzungen enthaltend mindestens ein Aldimin der Formel (I) bestehen aus einer Komponente **L1,** die mindestens ein Polyisocyanat und/oder mindestens ein Isocyanat-haltiges Polyurethanpolymer **P** umfasst, und einer Komponente **L2**, die mindestens ein Aldimin der Formel (I) sowie mindestens ein Polyol und/oder mindestens ein Polyamin umfasst. Die Komponente **L1** entspricht dabei der oben beschriebenen Komponente **K1,** ausser dass sie keine Aldimin-haltige Verbindung **AV** enthalten muss; in der gleichen Weise entspricht auch die Komponente **L2** der oben beschriebenen Komponente **K2**. Folglich entsprechen die als Polyisocyanat, Isocyanat-haltiges Polyurethanpolymer **P**, Polyol und Polyamin geeigneten Substanzen jenen, wie sie für die zweikomponentige Isocyanat-haltige Zusammensetzung bestehend aus den Komponenten **K1** und **K2** beschrieben wurden. Weiterhin kann die Zusammensetzung gegebenenfalls weitere Bestandteile enthalten, wobei dieselben Weichmacher, Lösemittel, Füllstoffe, Katalysatoren, Reaktivverdünner und Vernetzer, latente Polyamine, Trocknungsmittel, Rheologiemodifizierer, Haftvermittler, Stabilisatoren, oberflächenaktiven Substanzen und Biocide eingesetzt werden können, welche bereits für die die zweikomponentige Isocyanat-haltige Zusammensetzung bestehend aus den Komponenten **K1** und **K2** genannt wurden. Für solche zweikomponentigen Isocyanat-haltigen Zusammensetzungen bevorzugte Aldimine der Formel (I) sind solche, welche mehr als einen aktiven Wasserstoff pro Molekül aufweisen. Typischerweise ist das Aldimin der Formel (I) in einer Menge von 0.1 bis 50 Gewichts-%, bevorzugt 0.5 bis 30 Gewichts-%, und insbesondere 1 bis 20 Gewichts-%, bezogen auf die zweikomponentige Isocyanat-haltige Zusammensetzung, vorhanden.

Aufgrund der Geruchsfreiheit der Aldimine der Formel (I), deren Umsetzungsprodukten wie die vorgängig beschriebenen Aldimin-haltigen Verbindungen **AV** wie auch der bei der Hydrolyse dieser Substanzen freigesetzten Aldehyde **A** härten die beschriebenen Kunststoffvorläufer ohne Geruchsbildung aus. Sie können somit auch für Geruchsfreiheit voraussetzende Anwendungen eingesetzt werden, wie beispielsweise für Verklebungen, Abdichtungen, Beschichtungen oder Beläge im Innern von Fahrzeugen oder Gebäuden. Solche Anwendungen sind beispielsweise Klebstoffe, Dichtstoffe, Beschichtungen oder Bodenbeläge in der industriellen Fertigung oder Reparatur oder im Tief- oder Hochbau oder Innenausbau von Transportmitteln oder Bauwerken. Speziell erwähnt werden sollen Anwendungen als elastischer Klebstoff bei der Fertigung von Fahrzeugen zu Wasser oder zu Lande, insbesondere Automobile, Schiffe, Busse, Lastkraftwagen oder Züge, sowie Anwendungen als elastischer Dichtstoff bei der Fertigung von Transportmitteln oder Bauwerken.

**Beispiele**

Beschreibung der Messmethoden

Die **Infrarotspektren** wurden auf einem FT-IR Gerät 1600 von Perkin-Elmer gemessen (horizontale ATR-Messeinheit mit ZnSe-Kristall); die Proben wurden unverdünnt als Filme aufgetragen. Die Absorptionsbanden sind in Wellenzahlen ($cm^{-1}$) angegeben (Messfenster: 4000-650 $cm^{-1}$).

**$^1$H-NMR-Spektren** wurden gemessen bei 298K auf einem Spektrometer des Typs Bruker DPX-300 bei 300.13 MHz; die chemischen

Verschiebungen $\delta$ sind angegeben in ppm relativ zu Tetramethylsilan (TMS), Kopplungskonstanten *J* sind angegeben in Hz. Die Kopplungsmuster (t, m) wurden angegeben, auch wenn es sich nur um Pseudokopplungsmuster handelt.

Die **Viskosität** wurde auf einem thermostatisierten Kegel-Platten-Viskosimeter Physica UM (Kegeldurchmesser 20 mm, Kegelwinkel 1°, Kegelspitze-Platten-Abstand 0.05 mm, Schergeschwindigkeit 10 bis 1000 $s^{-1}$) gemessen.

Der totale **Gehalt an Aldiminogruppen und freien Aminogruppen** in den hergestellten Verbindungen ("Amin-Gehalt") wurde titrimetrisch bestimmt (mit 0.1N $HClO_4$ in Eisessig, gegen Kristallviolett) und ist stets angegeben in mmol $NH_2$/g (auch wenn es sich nicht nur um primäre Aminogruppen handelt).

Aldimine der Formel (I) enthaltend einen freien Wasserstoff

**Beispiel 1 (Aldimin *AL1)***

In einem Rundkolben wurden unter Stickstoffatmosphäre 40.64 g (0.143 mol) 2,2-Dimethyl-3-lauroyloxy-propanal vorgelegt. Unter kräftigem Rühren wurden aus einem Eintropftrichter innerhalb von 5 Minuten 11.68 g (0.133 mol) N-Methyl-1,3-propandiamin zugegeben, wobei die Temperatur des Reaktionsgemisches auf 38°C anstieg. Danach wurden die flüchtigen Bestandteile im Vakuum entfernt (10 mbar, 80°C). Man erhielt 49.8 g einer farblosen, bei Raumtemperatur dünnflüssigen, klaren und geruchlosen Flüssigkeit, die einen Amin-Gehalt von 5.20 mmol $NH_2$/g aufwies.

Das Produkt liegt grösstenteils in der offenkettigen (Aldimin-) Form vor.
IR:3329(N-H),2954sh,2922,2852,789,1736(C=O),1668(C=N),1466,1419sh,1392,1374, 1348, 1300, 1249, 1234, 1160, 1112, 1069, 1058, 1021, 996, 938, 886, 876, 820, 722. $^1$H-NMR ($CDCl_3$, 300 K): δ 7.53 (*s*, 1 H, CH=N), 4.01 (s, 2 H, $CH_2O$), 3.44 (*t*, 2 H, CH=NC*H*$_2$CH$_2$), 2.58 (*t*, 2 H, NHC*H*$_2$), 2.42 (s, 3 H, C*H*$_3$NH), 2.30 (*t,* 2 H, $CH_2CO$), 1.76 (*t*, 2 H, CH=NCH$_2$C*H*$_2$),1.61 (*m*, 3 H, $CH_2CH_2CO$ und $CH_3$N*H*$CH_2$), 1.27 (*m*, 16 H, $CH_3$-(C*H*$_2$)$_8$-$CH_2CH_2CO$), 1.10 (s, 6 H, C (C*H*$_3$)$_2$-$CH_2O$), 0.89 (*t*, 3 H, C*H*$_3$-$(CH_2)_{10}$-CO).

**Beispiel 2 (Aldimin AL2)**

In einem Rundkolben wurden unter Stickstoffatmosphäre 30.13 g (0.106 mol) 2,2-Dimethyl-3-lauroyloxy-propanal vorgelegt. Unter kräftigem Rühren wurden aus einem Eintropftrichter innerhalb von 5 Minuten 15.00 g (0.096 mol) N-Cyclohexyl-1,3-propandiamin zugegeben, wobei die Temperatur des Reaktionsgemisches auf 36˚C anstieg. Danach wurden die flüchtigen Bestandteile im Vakuum entfernt (10 mbar, 80˚C). Man erhielt 43.2 g einer farblosen, bei Raumtemperatur dünnflüssigen, klaren und geruchlosen Flüssigkeit, die einen Amin-Gehalt von 4.39 mmol $NH_2$/g aufwies. Das Produkt liegt grösstenteils in der offenkettigen (Aldimin-) Form vor. IR: 3308 (N-H), 2921, 2851, 2659, 1737 (C=O), 1668 (C=N), 1465, 1449, 1418sh,
1393, 1366, 1346, 1301, 1248, 1158, 1111, 1068, 1020, 1002, 938, 888, 845, 797, 721. $^1$H-NMR ($CDCl_3$, 300 K): δ 7.53 *(s,* 1 H, CH=N), 4.01 *(s,* 2 H, $CH_2O$), 3.43 *(t,* 2 H, CH=NC*H*$_2$CH$_2$), 2.65 *(t,* 2 H, NHC*H*$_2$), 2.40 (*s*, 1 H, Cy-C$^1$*H*NH), 2.29 *(t,* 2 H, $CH_2CO$), 1.86 *(m,* 2 H, 2 Cy-H), 1.72 *(m,* 4 H, 2 Cy-H und CH=NCH$_2$C*H*$_2$), 1.60 *(m,* 3 H, C*H*$_2$CH$_2$CO und $CH_3$N*H*$CH_2$), 1.26 *(m,* 22 H, $CH_3$-(C*H*$_2$)$_8$-$CH_2CH_2CO$ und 6 Cy-H), 1.09 (s, 6 H, C(C*H*$_3$)$_2$-$CH_2O$), 0.88 *(t,* 3 H, C*H*$_3$-$(CH_2)_{10}$-CO).

**Beispiel 3 (Aldimin *AL3)***

In einem Rundkolben wurden unter Stickstoffatmosphäre 69.31 g (0.244 mol) 2,2-Dimethyl-3-lauroyloxy-propanal vorgelegt. Unter kräftigem Rühren wurden aus einem Eintropftrichter innerhalb von 5 Minuten 14.72 g (0.112 mol) Dipropylentriamin zugegeben, wobei die Temperatur des Reaktionsgemisches auf 36˚C anstieg. Danach wurden die flüchtigen Bestandteile im Vakuum entfernt (10 mbar, 80˚C). Man erhielt 79.7 g einer farblosen, bei Raumtemperatur dünnflüssigen, klaren und geruchlosen Flüssigkeit, die einen Amin-Gehalt von 4.17 mmol $NH_2$/g aufwies. Das Produkt liegt grösstenteils in der offenkettigen (Aldimin-) Form vor.
IR:3308(N-H), 2952sh, 2921,2851,1737(C=O), 1667(C=N), 1466, 1418sh, 1393, 1373, 1348, 1301, 1248, 1234, 1159, 1111, 1070, 1019, 1001, 936, 875, 722.
$^1$H-NMR ($CDCl_3$, 300 K): δ 7.53 (*s*, 2 H, CH=N), 4.01 *(s,* 4 H, $CH_2O$), 3.42 *(t,* 4 H, CH=NC*H*$_2$CH$_2$), 2.61 *(t,* 4 H, NHC*H*$_2$), 2.29 (*t*, 4 H, $CH_2CO$), 1.73 *(m,* 4 H, CH=NCH$_2$C*H*$_2$), 1.59 *(m,* 5 H, C*H*$_2$CH$_2$CO und $CH_2$N*H*$CH_2$), 1.25 *(m,* 32 H, $CH_3$-(C*H*$_2$)$_8$-$CH_2CH_2CO$), 1.09 *(s,* 12 H, C(C*H*$_3$)$_2$-$CH_2O$), 0.87 (*t*, 6 H, C*H*$_3$-$(CH_2)_{10}$-CO).

**Beispiel 4 (Aldimin *AL4)***

In einem Rundkolben wurden unter Stickstoffatmosphäre 34.15 g (0.120 mol) 2,2-Dimethyl-3-lauroyloxy-propanal vorgelegt. Unter kräftigem Rühren wurden aus einem Eintropftrichter innerhalb von 5 Minuten 12.02 g (0.056 mol) Bis-hexamethylentriamin (BHMT-HP; Invista) zugegeben, wobei die Temperatur des Reaktionsgemisches auf 35˚C anstieg. Danach wurden die flüchtigen Bestandteile im Vakuum entfernt (10 mbar, 80˚C). Man erhielt 43.6 g einer farblosen, bei Raumtemperatur dünnflüssigen, klaren und geruchlosen Flüssigkeit, die einen Amin-Gehalt von 3.68 mmol $NH_2$/g aufwies. Das Produkt liegt grösstenteils in der offenkettigen (Aldimin-) Form vor.
IR: 2922, 2851, 1737 (C=O), 1668 (C=N), 1465, 1417, 1393, 1373, 1340, 1248, 1234, 1159, 1111, 1020, 1003, 933, 870, 722.
$^1$H-NMR ($CDCl_3$, 300 K): δ 7.52 (s, 2 H, CH=N), 4.02 (*s*, 4 H, $CH_2O$), 3.36 (*t*, 4 H, CH=NC*H*$_2$CH$_2$), 2.59 (*t*, 4 H, NHC*H*$_2$), 2.29 (*t*, 4 H, $CH_2CO$), 1.76-1.51 (*m*, 13 H, CH=NCH$_2$C*H*$_2$, NHCH$_2$C*H*$_2$, C*H*$_2$CH$_2$CO und $CH_2$N*H*$CH_2$), 1.27 (*m*, 40 H, $CH_3$-(C*H*$_2$)$_8$-$CH_2CH_2CO$ und NHCH$_2$CH$_2$C*H*$_2$), 1.10 (s, 12 H, C(C*H*$_3$)$_2$-$CH_2O$), 0.88 (*t*, 6 H, C*H*$_3$-$(CH_2)_{10}$-CO).

**Beispiel 5 (Aldimin *AL5)***

In einem Rundkolben wurden unter Stickstoffatmosphäre 30.28 g (0.106 mol) 2,2-Dimethyl-3-lauroyloxy-propanal vorgelegt. Unter kräftigem Rühren wurden aus einem Eintropftrichter innerhalb von 5 Minuten 5.00 g (0.049 mol) Diethylentriamin zugegeben. Danach wurden die flüchtigen Bestandteile im Vakuum entfernt (10 mbar, 80˚C). Man erhielt 33.1 g einer farblosen, bei Raumtemperatur dünnflüssigen, klaren und geruchlosen Flüssigkeit, die einen Amin-Gehalt von 4.07 mmol $NH_2$/g aufwies. Das Produkt liegt grösstenteils in der offenkettigen (Aldimin-) Form vor.
IR: 3348 (N-H), 2952, 2921, 2852, 1735 (C=O), 1668 (C=N), 1632, 1465, 1417, 1393, 1373, 1345, 1248, 1232, 1158,

1110, 1056, 1022, 1005, 986, 931, 903, 875, 820, 721.

**Beispiel 6 (Aldimin AL6)**

In einem Rundkolben wurden unter Stickstoffatmosphäre 20.97 g (0.074 mol) 2,2-Dimethyl-3-lauroyloxy-propanal vorgelegt. Unter kräftigem Rühren wurden aus einem Eintropftrichter innerhalb von 5 Minuten 10.00 g (0.067 mol) Triethylenglykol-monoamin (Jeffamine® XTA-250; Huntsman) zugegeben, wobei die Temperatur des Reaktionsgemisches auf 33°C anstieg. Danach wurden die flüchtigen Bestandteile im Vakuum entfernt (10 mbar, 80°C). Man erhielt 29.5 g einer farblosen, bei Raumtemperatur dünnflüssigen, klaren und geruchlosen Flüssigkeit, die einen Amin-Gehalt von 2.21 mmol $NH_2$/g aufwies. Das Produkt liegt grösstenteils in der offenkettigen (Aldimin-) Form vor. IR: 3444br (O-H), 2952sh, 2921, 2852, 1736 (C=O), 1668 (C=N), 1466, 1418, 1394, 1374, 1366, 1350, 1301sh, 1248, 1145sh, 1116, 1067, 1023sh, 998sh, 932, 890, 829, 722.
$^1$H-NMR ($CDCl_3$, 300 K): δ 7.59 (*s*, 1 H, CH=N), 4.03 (s, 2 H, $CH_2O$), 3.79-3.59 (*m*, 12 H, $HOCH_2CH_2OCH_2CH_2OCH_2CH_2N$), 3.47 (*s*, 1 H, $HOCH_2$), 2.31 (*t*, 2 H, $CH_2CO$), 1.61 (*m*, 2 H, $CH_2CH_2CO$), 1.27 (*m*, 16 H, $CH_3$-$(CH_2)_8$-$CH_2CH_2CO$), 1.11 (*s*, 6 H, $C(CH_3)_2$-$CH_2O$), 0.87 (*t*, 3 H, $CH_3$-$(CH_2)_{10}$-CO).

**Beispiel 7 (Aldimin *AL7*)**

In einem Rundkolben wurden unter Stickstoffatmosphäre 34.48 g (0.121 mol) 2,2-Dimethyl-3-lauroyloxy-propanal vorgelegt. Unter kräftigem Rühren wurden aus einem Eintropftrichter innerhalb von 15 Minuten 20.00 g (0.117 mol) Isophorondiamin (Vestamin® IPD, Degussa) zugegeben. Danach wurden die flüchtigen Bestandteile im Vakuum entfernt (10 mbar, 80°C). Zum so erhaltenen klaren, farblosen Öl gab man bei Raumtemperatur 25.25 g (0.121 mol) Isobornylacrylat (SR-506, Sartomer). Man liess 30 Minuten bei Raumtemperatur rühren, erwärmte die Mischung dann auf 85°C und hielt sie während 24 Stunden bei dieser Temperatur. Darauf wurden die flüchtigen Bestandteile im Hochvakuum entfernt (100°C). Man erhielt 72.0 g einer farblosen, bei Raumtemperatur dünnflüssigen, klaren und geruchlosen Flüssigkeit, die einen Amin-Gehalt von 3.09 mmol $NH_2$/g aufwies. Das Produkt liegt grösstenteils in der offenkettigen (Aldimin-) Form vor.
IR: 3322 (N-H), 2950, 2923, 2871, 2852, 1732 (C=O), 1668 (C=N), 1457, 1418sh, 1388sh, 1377, 1364, 1310, 1294, 1248, 1196, 1165, 1110, 1053, 1015, 987, 969, 942, 931 sh, 914, 893, 863, 840, 796, 722.

**Beispiel 8 (Aldimin *AL8)* (Vergleich)**

In einem Rundkolben wurden unter Stickstoffatmosphäre 48.18 g (0.243 mol) 3-Phenoxybenzaldehyd vorgelegt. Unter kräftigem Rühren wurden aus einem Eintropftrichter innerhalb von 5 Minuten 20.00 g (0.227 mol) N-Methyl-1,3-propandiamin zugegeben, wobei die Temperatur des Reaktionsgemisches auf 40°C anstieg. Danach wurden die flüchtigen Bestandteile im Vakuum entfernt (10 mbar, 80°C). Man erhielt 63.7 g einer hellgelben, bei Raumtemperatur dünnflüssigen, klaren und stark riechenden Flüssigkeit, die einen Amin-Gehalt von 7.08 mmol $NH_2$/g aufwies. Das Produkt liegt mehrheitlich in der cyclischen (Tetrahydropyrimidin-) Form vor.
IR: 3270 (N-H), 3060, 3036, 2978, 2940, 2837, 2773, 2692, 1935, 1865, 1778, 1702, 1645, 1582, 1483, 1456, 1442, 1418, 1370, 1353, 1308, 1236, 1210, 1188, 1163, 1128, 1108, 1072, 1053, 1023, 990, 964, 937, 917, 900, 889, 877, 839, 775, 748, 690. $^1$H-NMR ($CDCl_3$, 300 K): δ 7.42-7.28 (m, 5 Ar-H), 7.16-7.01 (m, 4 Ar-H), 3.74 (*s*, 1 H, Ar-C*H*(NH)N), 3.14 (*m*, 2 H, $HNCH^{eq}H^{ax}$ und $CH_3NCH^{eq}H^{ax}$), 2.78 (*m*, 1 H, $HNCH^{eq}H^{ax}$), 2.35 (m, 1 H, $CH_3NCH^{eq}H^{ax}$), 2.06 (*s*, 3 H, $CH_3N$), 1.90 (*m*, 1 H, $CH_3NCH_2CH^{eq}H^{ax}$), 1.58 (*m*, 2 H, $CH_3NCH_2CH^{eq}H^{ax}$ und $HNCH_2$).

**Beispiel 9 (Aldimin *AL9)***

In einem Rundkolben wurden unter Stickstoffatmosphäre 39.21 g (0.138 mol) 2,2-Dimethyl-3-lauroyloxy-propanal vorgelegt. Unter kräftigem Rühren wurden aus einem Eintropftrichter innerhalb von 10 Minuten 10.00 g (0.135 mol) 1,3-Diaminopropan zugegeben, wobei die Temperatur des Reaktionsgemisches auf 44°C anstieg. Danach wurden die flüchtigen Bestandteile im Vakuum entfernt (10 mbar, 70°C). Zum so erhaltenen klaren, farblosen Öl gab man bei 70°C 24.20 g (0.141 mol) Diethylmaleinat, liess während einer Stunde rühren, erhöhte die Temperatur auf 100°C und liess weitere drei Stunden rühren. Darauf wurden die flüchtigen Bestandteile im Hochvakuum entfernt (70°C). Man erhielt 68.0 g einer hellgelben, bei Raumtemperatur dünnflüssigen, klaren und geruchlosen Flüssigkeit, die einen Amin-Gehalt von 3.54 mmol $NH_2$/g aufwies. Das Produkt liegt grösstenteils in der offenkettigen (Aldimin-) Form vor.
IR: 3325 (N-H), 2953sh, 2923, 2852, 1732 (C=O), 1668 (C=N), 1466, 1418, 1392, 1368, 1345, 1296, 1256, 1225, 1173sh, 1154, 1112, 1029, 982, 933, 858, 774, 722.

**Beispiel 10 (Aldimin *AL10*)**

In einem Rundkolben wurden unter Stickstoffatmosphäre 34.48 g (0.121 mol) 2,2-Dimethyl-3-lauroyloxy-propanal vorgelegt. Unter kräftigem Rühren wurden aus einem Eintropftrichter innerhalb von 15 Minuten 20.00 g (0.117 mol) Isophorondiamin (Vestamin® IPD, Degussa) zugegeben. Danach wurden die flüchtigen Bestandteile im Vakuum entfernt (10 mbar, 80°C). Zum so erhaltenen klaren, farblosen Öl gab man bei 70°C 21.30 g (0.124 mol) Diethylmaleinat und liess während 6 Stunden rühren. Darauf wurden die flüchtigen Bestandteile im Hochvakuum entfernt (70°C). Man erhielt 64.6 g einer farblosen, bei Raumtemperatur dünnflüssigen, klaren und geruchlosen Flüssigkeit, die einen Amin-Gehalt von 3.38 mmol $NH_2$/g aufwies. Das Produkt liegt grösstenteils in der offenkettigen (Aldimin-) Form vor.
IR: 3320 (N-H),2948sh,2926,2852,1734(C=O),1667 (C=N), 1463, 1418sh, 1367, 1349, 1296, 1253, 1175, 1158, 1112, 1098sh, 1028, 983, 930, 893, 860, 800, 774, 751, 722.

**Beispiel 11 (Aldimin *AL11*)**

In einem Rundkolben wurden unter Stickstoffatmosphäre 40.50 g (0.142 mol) 2,2-Dimethyl-3-lauroyloxy-propanal vorgelegt. Unter kräftigem Rühren wurden aus einem Eintropftrichter innerhalb von 10 Minuten 10.00 g (0.133 mol) 3-Amino-1-propanol zugegeben. Danach wurden die flüchtigen Bestandteile im Vakuum entfernt (10 mbar, 80°C). Man erhielt 47.9 g einer farblosen, bei Raumtemperatur dünnflüssigen, klaren und geruchlosen Flüssigkeit, die einen Amin-Gehalt von 2.74 mmol $NH_2$/g aufwies. Das Produkt liegt mehrheitlich (ca. 94% gemäss $^1$H-NMR) in der cyclischen (Tetrahydrooxazin-) Form vor. IR: 3322 (N-H), 2953sh, 2922, 2852, 1735 (C=O), 1668 (w, C=N), 1465, 1431, 1418, 1394, 1376, 1339sh, 1256sh, 1234, 1219, 1168sh, 1149, 1111, 1064, 1019, 988, 950, 906,878,853,800,762,722.
$^1$H-NMR ($CDCl_3$, 300 K): δ 4.10-3.89 *(m,* 4 H), 3.70 *(t,* 1 H), 3.17 *(m,* 1 H), 2.88 *(t,* 1 H), 2.32 (*t*, 2 H, $CH_2CO$), 1.68 *(m,* 5 H), 1.27 *(m,* 16 H, $CH_3$-($CH_2$)$_8$-$CH_2CH_2CO$), 0.95 *(s,* 6 H, C($CH_3$)$_2$-$CH_2O$), 0.88 (*t*, 3 H, $CH_3$-($CH_2$)$_{10}$-CO).

**Beispiel 12 (Aldimin *AL12*)**

In einem Rundkolben wurden 10.00 g (0.088 mol) Cysteamin Hydrochlorid in 10 ml Wasser gelöst, unter Stickstoffatmosphäre mit 25.79 g (0.091 mol) 2,2-Dimethyl-3-lauroyloxy-propanal versetzt und gut gerührt. Die organischen Bestandteile wurden mit Ethylacetat extrahiert, über $MgSO_4$ getrocknet und die flüchtigen Bestandteile im Vakuum entfernt (10 mbar, 80°C). Man erhielt 30.9 g einer farblosen, bei Raumtemperatur dünnflüssigen, klaren und geruchlosen Flüssigkeit, die einen Amin-Gehalt von 2.84 mmol $NH_2$/g aufwies. Das Produkt liegt mehrheitlich (ca. 93% gemäss $^1$H-NMR) in der cyclischen (Thiazolidin-) Form vor.
IR: 3317 (N-H), 2954, 2921, 2852, 1732 (C=O), 1667 (w, C=N), 1466, 1418, 1393, 1375,1351 sh,1317,1248, 1233, 1161, 1108, 1076, 1011, 989sh, 920, 828, 792sh, 721.
$^1$H-NMR ($CDCl_3$, 300 K): δ4.54 (*s*, 1H, HNC*H*(C)S), 4.05 (*s*, 2 H, $CH_2O$), 3.54 (*m*, 1 H von HNC$H_2$C$H_2$S), 2.91 (*m*, 2 H von HNC$H_2$$CH_2$S), 2.78 (*m*, 1 H von HNC$H_2$C$H_2$S), 2.30 (*t*, 2 H, $CH_2CO$), 1.89 (*s*, 1 H, *H*N$CH_2$), 1.62 (*m*, 2 H, C$H_2$$CH_2CO$), 1.28 (*m*, 16 H, $CH_3$-(C$H_2$)$_8$-$CH_2CH_2CO$), 1.07 (*s*, 6 H, C(C$H_3$)$_2$-$CH_2O$), 0.87 (*t*, 3H, C$H_3$-($CH_2$)$_{10}$-CO).

**Beispiel 13 (Aldimin *AL13*)**

In einem Rundkolben wurden unter Stickstoffatmosphäre 28.06 g (0.099 mol) 2,2-Dimethyl-3-lauroyloxy-propanal vorgelegt. Unter kräftigem Rühren wurden aus einem Eintropftrichter innerhalb von 3 Minuten 10.00 g (0.095 mol) 2-(2-Aminoethoxy)-ethanol (Diglycolamine® Agent; Huntsman) zugegeben, wobei die Temperatur des Reaktionsgemisches auf 40°C anstieg. Danach wurden die flüchtigen Bestandteile im Vakuum entfernt (10 mbar, 80°C). Man erhielt 36.3 g einer farblosen, bei Raumtemperatur dünnflüssigen, klaren und geruchlosen Flüssigkeit, die einen Amin-Gehalt von 2.58 mmol $NH_2$/g aufwies. Das Produkt liegt grösstenteils in der offenkettigen (Aldimin-) Form vor.
IR: 3435br (O-H), 2954sh, 2922, 2852, 1736 (C=O), 1668 (C=N), 1466, 1418, 1394, 1375, 1248, 1233, 1160, 1127, 1062, 1022, 933, 893, 813, 721.
$^1$H-NMR ($CDCl_3$, 300 K): δ 7.59 (*s*, 1 H, CH=N), 4.03 (*s*, 2 H, $CH_2O$), 3.71 (*m*, 4 H, HO$CH_2$C$H_2$OC$H_2$$CH_2$N), 3.58 (*m*, 4 H, HOC$H_2$$CH_2$O$CH_2$C$H_2$N), 2.44 (br *s*, 1 H, *H*O$CH_2$), 2.30 (*t,* 2 H, $CH_2CO$), 1.61 (*m*, 2 H, C$H_2$$CH_2CO$), 1.26 (*m*, 16 H, $CH_3$-(C$H_2$)$_8$-$CH_2CH_2CO$), 1.11 (*s*, 6 H, C(C$H_3$)$_2$-$CH_2O$), 0.88 (*t,* 3 H, C$H_3$-($CH_2$)$_{10}$-CO).

**Beispiel 14 (Aldimin *AL14*)**

In einem Rundkolben wurden unter Stickstoffatmosphäre 34.51 g (0.121 mol) 2,2-Dimethyl-3-lauroyloxy-propanal vorgelegt. Unter kräftigem Rühren wurden aus einem Eintropftrichter innerhalb von 5 Minuten 33.39 g N-Oleyl-1,3-propandiamin (Duomeen® O, Akzo Nobel; Aminzahl = 337 mg KOH/g) zugegeben, wobei die Temperatur des Reaktionsgemisches auf 48°C anstieg. Danach wurden die flüchtigen Bestandteile im Vakuum entfernt (10 mbar, 80°C).

Man erhielt 65.7 g einer farblosen, bei Raumtemperatur dünnflüssigen, klaren und geruchlosen Flüssigkeit, die einen Amin-Gehalt von 3.07 mmol $NH_2$/g aufwies. Das Produkt liegt grösstenteils in der offenkettigen (Aldimin-) Form vor. IR: 3307 (N-H), 3001sh, 2954sh, 2921, 2851, 1739 (C=O), 1668 (C=N), 1464, 1393,1375,1347,1301,1248,1158, 1114, 1067, 1020, 1000, 968, 935, 889, 721.

$^1$H-NMR ($CDCl_3$, 300 K): δ 7.53 *(t, J* = 1.2) und 7.51 (s) (total 1 H (Verhältnis ca. 0.85 / 0.15), CH=N), 5.34 (*m*, 2 H, $CH_2CH{=}CHCH_2$), 4.01 (*s*, 2 H, $CH_2O$), 3.43 (*t*, 2 H, $CH{=}NCH_2CH_2$), 2.60 (*m*, 4 H, $CH{=}NCH_2CH_2CH_2$ und $NHCH_2$), 2.30 (*t*, 2 H, $CH_2CO$), 2.01 (*m*, 4 H, $CH_2CH{=}CHCH_2$), 1.75 (*m*, 2 H, $CH{=}NCH_2CH_2$), 1.60 (*m*, 3 H, $CH_2CH_2CO$ und $CH_2NHCH_2$), 1.47 (*m*, 2 H, $CH_2NHCH_2CH_2$), 1.26 (*m*, 38 H, übrige $CH_2$-Gruppen), 1.09 (*s*, 6 H, $C(CH_3)_2$-$CH_2O$), 0.88 (*t*, 6 H, beide $CH_3CH_2CH_2$).

**Beispiel 15 (Aldimin *AL15)***

In einem Rundkolben wurden unter Stickstoffatmosphäre 40.00 g (0.141 mol) 2,2-Dimethyl-3-lauroyloxy-propanal vorgelegt. Unter kräftigem Rühren wurden aus einem Eintropftrichter innerhalb von 5 Minuten 24.00 g (0.128 mol) N-(2-Ethylhexyl)-1,3-propandiamin (BASF) zugegeben, die Mischung auf 80˚C erwärmt und gleichzeitig die flüchtigen Bestandteile im Vakuum entfernt (10 mbar). Man erhielt 61.5 g einer farblosen, bei Raumtemperatur dünnflüssigen, klaren und geruchlosen Flüssigkeit, die einen Amin-Gehalt von 4.12 mmol $NH_2$/g aufwies. Das Produkt liegt grösstenteils in der offenkettigen (Aldimin-) Form vor.

IR: 3322(N-H),2955,2922,2870sh,2852,2824sh, 1738 (C=O), 1668 (C=N), 1464, 1393, 1376,1342,1300, 1248, 1235, 1157, 1114, 1069, 1020, 1000, 935, 894, 873, 766, 723.

**Beispiel 16 (Aldimin *AL16)***

In einem Rundkolben wurden unter Stickstoffatmosphäre 40.00 g (0.141 mol) 2,2-Dimethyl-3-lauroyloxy-propanal vorgelegt. Unter kräftigem Rühren wurden aus einem Eintropftrichter innerhalb von 5 Minuten 33.19 g destilliertes N-Cocoalkyl-1,3-propandiamin (Duomeen® CD, Akzo Nobel; Aminzahl = 432 mg KOH/g) zugegeben, die Mischung auf 80˚C erwärmt und gleichzeitig die flüchtigen Bestandteile im Vakuum entfernt (10 mbar). Man erhielt 70.7 g eines weissen, bei Raumtemperatur festen und geruchlosen Körpers, der einen Amin-Gehalt von 3.62 mmol $NH_2$/g aufwies. Das Produkt liegt grösstenteils in der offenkettigen (Aldimin-) Form vor.

IR: 3314 (N-H),2953,2919,2851,2815sh, 1738 (C=O), 1668 (C=N), 1465, 1393, 1375,1349,1301,1248,1234,1159, 1113, 1070, 1020, 1002, 935, 891, 872, 721.

**Beispiel 17 (Aldimin *AL17)***

In einem Rundkolben wurden unter Stickstoffatmosphäre 30.00 g (0.105 mol) 2,2-Dimethyl-3-lauroyloxy-propanal vorgelegt. Unter kräftigem Rühren wurden innerhalb von 5 Minuten portionenweise 35.81 g N-($C_{16-22}$-Alkyl)-1,3-propandiamin (Duomeen® M, Akzo Nobel; Aminzahl = 301 mg KOH/g) zugegeben, die Mischung auf 80˚C erwärmt und gleichzeitig die flüchtigen Bestandteile im Vakuum entfernt (10 mbar). Man erhielt 65.5 g eines weissen, bei Raumtemperatur festen und geruchlosen Körpers, der einen Amin-Gehalt von 2.99 mmol $NH_2$/g aufwies. Das Produkt liegt grösstenteils in der offenkettigen (Aldimin-) Form vor.

IR: 3314(N-H),2952sh,2917,2849,2812sh,1739 (C=O), 1668 (C=N), 1464, 1393, 1375, 1368,1349,1301,1248,1233, 1159,1128sh,1114,1069, 1020, 1000, 936, 890, 871, 720.

**Beispiel 18 (Aldimin *AL18)***

In einem Rundkolben wurden unter Stickstoffatmosphäre 35.00 g (0.123 mol) 2,2-Dimethyl-3-lauroyloxy-propanal vorgelegt. Unter kräftigem Rühren wurden innerhalb von 5 Minuten 36.31 g 50˚C warmes N-Talgalkyl-1,3-propandiamin (Duomeen® T, Akzo Nobel; Aminzahl = 346 mg KOH/g) zugegeben, die Mischung auf 80˚C erwärmt und gleichzeitig die flüchtigen Bestandteile im Vakuum entfernt (10 mbar). Man erhielt 69.2 g eines schmutzigweissen, bei Raumtemperatur festen und geruchlosen Körpers, der einen Amin-Gehalt von 3.20 mmol $NH_2$/g aufwies. Das Produkt liegt grösstenteils in der offenkettigen (Aldimin-) Form vor.

IR: 3316 (N-H), 2954sh, 2919, 2851, 2815sh, 1739 (C=O), 1668 (C=N), 1464, 1393, 1375, 1347, 1300, 1248, 1233, 1158, 1128sh, 1114, 1068, 1021, 1000, 968, 936, 917sh, 889, 873, 721.

Aldimine der Formel (I) enthaltend mehr als einen freien Wasserstoff

**Beispiel 19 (Aldimin *AL19*)**

In einem Rundkolben wurde unter Stickstoffatmosphäre 33.93g(119 mmol) 2,2-Dimethyl-3-lauroyloxy-propanal vorgelegt. Unter kräftigem Rühren wurden aus einem Eintropftrichter innerhalb von 5 Minuten 10.00g (57mmol) N,N'-Bis-(3-aminopropyl)-ethylendiamin (N4-Amin,BASF) zugegeben, wobei die Temperatur des Reaktionsgemisches auf 40°C anstieg. Danach wurden die flüchtigen Bestandteile im Vakuum entfernt (10 mbar, 80°C). Man erhielt 41.7 g einer farblosen, bei Raumtemperatur dünnflüssigen, klaren und geruchlosen Flüssigkeit, die einen Amin-Gehalt von 5.13 mmol $NH_2$/g aufwies. Das Produkt liegt grösstenteils in der offenkettigen (Aldimin-) Form vor.
IR: 3306(N-H),2954sh,2922,2852,2826sh,1736(C=O),1667(C=N),1465,1419 sh, 1393, 1373, 1345, 1301, 1249, 1158, 1112, 1068, 1020, 997, 936, 869, 722.
$^1$H-NMR ($CDCl_3$, 300 K): δ 7.53 *(t, J* = 1.2, 2 H, CH=N), 4.01 (*s*, 4 H, $CH_2O$), 3.43 (*t*, 4 H, CH=NC$H_2$$CH_2$), 2.70 (*s*, 4 H, NHC$H_2$C$H_2$NH), 2.63 (*t*, 4 H, CH=N$CH_2$$CH_2$C$H_2$NH), 2.30 (*t*, 4 H, $CH_2$CO), 1.75 (*m*, 4 H, CH=N$CH_2$C$H_2$), 1.60 (*m*, 6 H, C$H_2$$CH_2$CO und $CH_2$N*H*$CH_2$), 1.26 (*m*, 32 H, $CH_3$-(C$H_2$)$_8$-$CH_2$$CH_2$CO), 1.09 (*s*, 12 H, C(C$H_3$)$_2$-$CH_2$0), 0.88 (*t*, 6 H, C$H_3$-($CH_2$)$_{10}$-CO).

**Beispiel 20 (Aldimin *AL20*)**

In einem Rundkolben wurden unter Stickstoffatmosphäre 30.00 g (0.105 mol) 2,2-Dimethyl-3-lauroyloxy-propanal vorgelegt. Unter kräftigem Rühren wurden mittels Pipette 10.00 g eines Polyethylenimins mit mittlerem Molekulargewicht 800 (Lupasol® FG, BASF) zugegeben, wobei die Temperatur des Reaktionsgemisches auf 46°C anstieg. Danach wurden die flüchtigen Bestandteile im Vakuum entfernt (10 mbar, 80°C). Man erhielt 38.1 g einer blassgelben, klaren und geruchlosen Flüssigkeit, die eine Viskosität bei 20°C von 1430 mPa·s und einen Amin-Gehalt von ca. 4.7 mmol $NH_2$/g aufwies. Das Produkt liegt grösstenteils in der offenkettigen (Aldimin-) Form vor.
IR: 3314br (N-H), 2952sh, 2922, 2851, 2814sh, 1735 (C=O), 1668 (C=N), 1632sh,
1464, 1419, 1373, 1346, 1249, 1232, 1158, 1112, 1053, 1022, 931, 915, 876, 722. $^1$H-NMR ($CDCl_3$, 300 K): δ 7.60 und 7.54 (2xs, total 1 H (Verhältnis ca. 1 / 2), CH=N), 4.01 und 4.00 (2×s, total 2 H (Verhältnis ca. 1 / 2), $CH_2O$), 3.52-3.44 (*m*, 2 H, CH=NC$H_2$$CH_2$), 3.0-2.5 (*m*, ca. 6.8 H, alle C$H_2$NC$H_2$), 2.30 (*t*, 2 H, $CH_2$CO), 1.91 (br *s*, ca. 1 H, $CH_2$N*H*), 1.61 (*m*, 2 H, C$H_2$$CH_2$CO), 1.26 (*m*, 16 H, $CH_3$-(C$H_2$)$_8$-$CH_2$$CH_2$CO), 1.10 und 1.09 (2×s, total 6 H (Verhältnis ca. 1 / 2), C (C$H_3$)$_2$-$CH_2$O), 0.88 (*t*, 3 H, C$H_3$-($CH_2$)$_{10}$-CO).

**Beispiel 21 (Aldimin *AL21*)**

In einem Rundkolben wurden unter Stickstoffatmosphäre 20.07 g (0.071 mol) 2,2-Dimethyl-3-lauroyloxy-propanal vorgelegt und mit 13.79 g einer 50-prozentigen wässrigen Lösung eines Polyethylenimins mit mittlerem Molekulargewicht 1300 (Lupasol® G20, BASF) versetzt. Die Mischung wurde unter Rühren auf 80°C erwärmt; danach wurden die flüchtigen Bestandteile im Vakuum entfernt (0.1 mbar, 80°C). Man erhielt 25.8 g einer blassgelben, klaren und geruchlosen Flüssigkeit, die eine Viskosität bei 20°C von 2060 mPa·s aufwies. Das Produkt liegt grösstenteils in der offenkettigen (Aldimin-) Form vor.
IR: 3306br (N-H), 2952, 2921, 2851, 2814sh, 1735 (C=O), 1667 (C=N), 1634, 1464, 1418sh, 1391sh, 1372, 1345, 1249, 1233, 1157, 1111, 1055, 1021, 931, 915sh, 875sh, 765, 744sh, 722.

**Beispiel 22 (Aldimin *AL22*)**

In einem Rundkolben wurden unter Stickstoffatmosphäre 20.00 g (0.070 mol) 2,2-Dimethyl-3-lauroyloxy-propanal vorgelegt und mit 14.45 g einer 50-prozentigen wässrigen Lösung eines Polyethylenimins mit mittlerem Molekulargewicht 2000 (Lupasol® G35, BASF) versetzt. Die Mischung wurde unter Rühren auf 80°C erwärmt; danach wurden die flüchtigen Bestandteile im Vakuum entfernt (0.1 mbar, 80°C). Man erhielt 26.1 g einer blassgelben, klaren und geruchlosen Flüssigkeit, die eine Viskosität bei 20°C von 2720 mPa·s aufwies. Das Produkt liegt grösstenteils in der offenkettigen (Aldimin-) Form vor.
IR: 3308br(N-H),2954sh,2921, 2851, 2814sh, 1735 (C=O), 1667 (C=N), 1633sh, 1464, 1419sh, 1372,1344,1249,1233,1157,1111,1052, 1022, 931, 915sh, 876sh, 762sh, 722.

**Beispiel 23 (Aldimin *AL23*)**

In einen Rundkolben wurden unter Stickstoffatmosphäre 15.36 g einer 50-prozentigen wässrigen Lösung eines Polyethylenimins mit mittlerem Molekulargewicht 750'000 (Lupasol® P, BASF), 26.52 g Ricinusöl (purum, Fluka) und

20.00 g (0.070 mol) 2,2-Dimethyl-3-lauroyloxy-propanal eingewogen. Die Mischung wurde unter Rühren auf 80˚C erwärmt; danach wurden die flüchtigen Bestandteile im Vakuum entfernt (0.1 mbar, 80˚C). Man erhielt 53.0 g einer hellgelben, klaren und geruchlosen Flüssigkeit, die eine Viskosität bei 20˚C von 18 Pa·s aufwies. Das Produkt liegt grösstenteils in der offenkettigen (Aldimin-) Form vor.
IR:3395br(O-H),3312(N-H),2954sh,2922,2851,1735(C=O),1667(C=N), 1632sh, 1464, 1418, 1372, 1348, 1238, 1156, 1111, 1052, 1022, 931, 914, 868, 722.

Umsetzungssprodukte der Aldimine der Formel (I) mit Verbindungen **D** (Aldimin-haltige Verbindungen **AV**)

**Beispiel 24 (Aldimin-haltige Verbindung *AP1*)**

In einem Rundkolben wurden unter Stickstoffatmosphäre 1.74 g (13.9 mmol NCO) 4,4'-Diphenylmethan-diisocyanat (MDI; Desmodur® 44 MC L, Bayer) vorgelegt und auf 50˚C erwärmt. Aus einem Eintropftrichter wurden unter gutem Rühren innerhalb von 5 Minuten 10.00 g (13.9 mmol) Aldimin *AL3* zugegeben und die Mischung während einer Stunde bei 50˚C gerührt. Man erhielt eine farblose, bei Raumtemperatur dickflüssige, klare und geruchlose Flüssigkeit mit einem Amin-Gehalt von 2.37 mmol $NH_2$/g, die auf einem angefeuchteten pH-Papier neutral reagierte.
IR: 3300 (N-H), 2952sh, 2922, 2851, 1735 (C=O), 1664 (C=N), 1647sh, 1595, 1527sh, 1513, 1466, 1416, 1395, 1375, 1305, 1244, 1215, 1196, 1162, 1112, 1056, 1018, 1000, 939, 918sh, 851, 813, 777, 751, 721.

**Beispiel 25 (Aldimin-haltige Verbindung *AP2*)**

In einem Rundkolben wurden unter Stickstoffatmosphäre 3.47 g (27.7 mmol NCO) 4,4'-Diphenylmethan-diisocyanat (MDI; Desmodur® 44 MC L, Bayer) vorgelegt und auf 50˚C erwärmt. Aus einem Eintropftrichter wurden unter gutem Rühren innerhalb von 5 Minuten 10.00 g (13.9 mmol) Aldimin ***AL3*** zugegeben und die Mischung während einer Stunde bei 50˚C gerührt. Man erhielt eine blassgelbe, bei Raumtemperatur dickflüssige, klare und geruchlose Flüssigkeit, die auf einem angefeuchteten pH-Papier neutral reagierte.
IR: 3308 (N-H), 2954sh, 2922, 2852, 2266 (N=C=O), 1735 (C=O), 1665 (C=N), 1596, 1526sh, 1514, 1467, 1415, 1395, 1374, 1306, 1244, 1216, 1197, 1162, 1110, 1059, 1018, 1000, 940, 918sh, 854, 813, 781, 751, 721.

**Beispiel 26 (Aldimin-haltige Verbindung *AP3)***

In einem Rundkolben wurden unter Stickstoffatmosphäre 12.94 g (103.4 mmol NCO) 4,4'-Diphenylmethan-diisocyanat (MDI; Desmodur® 44 MC L, Bayer) vorgelegt und auf 50˚C erwärmt. Aus einem Eintropftrichter wurden unter gutem Rühren innerhalb von 10 Minuten 42.16 g (51.7 mmol) Aldimin ***AL4*** zugegeben und die Mischung während einer Stunde bei 50˚C gerührt. Man erhielt eine hellgelbe, bei Raumtemperatur dickflüssige, klare und geruchlose Flüssigkeit, die auf einem angefeuchteten pH-Papier neutral reagierte.
IR: 3336 (N-H), 2922, 2852, 2265 (N=C=O), 1736 (C=O), 1666 (C=N), 1640, 1594, 1513, 1488, 1465, 1416, 1394, 1373, 1307, 1237, 1169, 1110, 1065, 1018, 1000sh, 932,918sh,848,812,776,754,723.

**Beispiel 27 (Aldimin-haltige Verbindung *AP4)***

In einem Rundkolben wurden unter Stickstoffatmosphäre 10.00 g (51.4 mmol NCO) 1,6-Hexamethylendüsocyanat-Trimerisat (Desmodur® N-3300, Bayer; NCO-Gehalt = 21.61 Gewichts-%) in 29.79 g trockenem Diisodecylphthalat (DIDP; Palatinol® Z, BASF) gelöst. Aus einem Eintropftrichter wurden unter gutem Rühren bei Raumtemperatur innerhalb von 10 Minuten 19.79 g (102.9 mmol) Aldimin ***AL1*** zugegeben und die Mischung während einer Stunde gerührt. Man erhielt eine farblose, bei Raumtemperatur dünnflüssige, klare und geruchlose Flüssigkeit mit einem Amin-Gehalt von 0.87 mmol $NH_2$/g, die auf einem angefeuchteten pH-Papier neutral reagierte.
IR: 3423 (N-H), 3326 (N-H), 2954, 2924, 2853, 1726 (C=O), 1688, 1650, 1600, 1579, 1529, 1462, 1377, 1335, 1272, 1164, 1121, 1072, 1039, 985, 965, 948, 764, 742, 704.

**Beispiel 28 (Aldimin-haltige Verbindung *AP5)***

In einem Rundkolben wurden unter Stickstoffatmosphäre 10.00 g (51.4 mmol NCO) 1,6-Hexamethylendiisocyanat-Trimerisat (Desmodur® N-3300, Bayer; NCO-Gehalt = 21.61 Gewichts-%) in 47.05 g trockenem Diisodecylphthalat (DIDP; Palatinol® Z, BASF) gelöst. Aus einem Eintropftrichter wurden unter gutem Rühren bei Raumtemperatur innerhalb von 10 Minuten 37.05 g (102.9 mmol) Aldimin ***AL3*** zugegeben und die Mischung während einer Stunde gerührt. Man erhielt eine farblose, bei Raumtemperatur dünnflüssige, klare und geruchlose Flüssigkeit mit einem Amin-Gehalt von 1.11 mmol $NH_2$/g, die auf einem angefeuchteten pH-Papier neutral reagierte.
IR: 3422 (N-H), 3308 (N-H), 2954, 2924, 2853, 1727 (C=O), 1689, 1651, 1600, 1579, 1528, 1462, 1377, 1334, 1272,

1161, 1121, 1072, 1039, 995, 948, 870, 764, 742, 704.

**Beispiel 29 (Aldimin-haltige Verbindung *AP6*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80˚C zu einem NCO-terminierten Polyurethanpolymeren mit einem Gehalt an freien Isocyanat-Gruppen von 1.86 Gewichts-% und einer Viskosität bei 20˚C von 24 Pa·s umgesetzt. Zu diesem Polymer wurden bei Raumtemperatur 8.51 g (22.1 mmol) Aldimin ***AL1*** gegeben und die Mischung mittels eines Zentrifugalmischers (SpeedMixer™ DAC 150, FlackTek Inc.) innig vermengt. Man erhielt eine klare, homogene und geruchlose Flüssigkeit mit einer Viskosität bei 20˚C von 40 Pa·s.

**Beispiel 30 (Aldimin-haltige Verbindung AP7)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80˚C zu einem NCO-terminierten Polyurethanpolymeren mit einem Gehalt an freien Isocyanat-Gruppen von 1.86 Gewichts-% und einer Viskosität bei 20˚C von 24 Pa·s umgesetzt. Zu diesem Polymer wurden bei Raumtemperatur 10.62 g (14.8 mmol) Aldimin ***AL3*** gegeben und die Mischung mittels eines Zentrifugalmischers (SpeedMixer™ DAC 150, FlackTek Inc.) innig vermengt. Man erhielt eine klare, homogene und geruchlose Flüssigkeit mit einer Viskosität bei 20˚C von 29 Pa·s.

**Beispiel 31 (Aldimin-haltige Verbindung *AP8*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80˚C zu einem NCO-terminierten Polyurethanpolymeren mit einem Gehalt an freien Isocyanat-Gruppen von 1.86 Gewichts-% und einer Viskosität bei 20˚C von 24 Pa·s umgesetzt. Zu diesem Polymer wurden bei Raumtemperatur 17.03 g (44.3 mmol) Aldimin ***AL1*** gegeben und die Mischung mittels eines Zentrifugalmischers (SpeedMixer™ DAC 150, FlackTek Inc.) innig vermengt. Nach 10 Minuten war die NCO-Bande im FT-IR-Spektrum (bei 2265 $cm^{-1}$) nicht mehr nachweisbar. Man erhielt eine klare, homogene und geruchlose Flüssigkeit mit einer Viskosität bei 20˚C von 52 Pa·s und einem Amin-Gehalt von 0.38 mmol $NH_2$/g.

**Beispiel 32 (Aldimin-haltige Verbindung *AP9*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80˚C zu einem NCO-terminierten Polyurethanpolymeren mit einem Gehalt an freien Isocyanat-Gruppen von 1.86 Gewichts-% und einer Viskosität bei 20˚C von 24 Pa·s umgesetzt. Zu diesem Polymer wurden bei Raumtemperatur 31.85 g (44.3 mmol) Aldimin ***AL3*** gegeben und die Mischung mittels eines Zentrifugalmischers (SpeedMixer™ DAC 150, FlackTek Inc.) innig vermengt. Nach 10 Minuten war die NCO-Bande im FT-IR-Spektrum (bei 2265 $cm^{-1}$) nicht mehr nachweisbar. Man erhielt eine klare, homogene und geruchlose Flüssigkeit mit einer Viskosität bei 20˚C von 44 Pa·s und einem Amin-Gehalt von 0.67 mmol $NH_2$/g.

**Beispiel 33 (Aldimin-haltige Verbindung *AP10)***

25.97 g (13.2 mmol OH) Polyoxypropylen-Diol (Acclaim® 4200 N, Bayer; OH-Zahl 28.5 mg KOH/g), 51.95 g (32.4 mmol OH) Polyol Caradol® MD34-02 (Polypropylenoxidpolyethylenoxid-Triol, OH-Zahl 35.0 mg KOH/g; Shell), 12.08 g (48.3 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80˚C zu einem NCO-terminierten Polyurethanpolymeren mit einem Gehalt an freien Isocyanat-Gruppen von 2.07 Gewichts-% und einer Viskosität bei 20˚C von 48 Pa·s umgesetzt. Zu diesem Polymer wurden bei Raumtemperatur 18.95 g (49.3 mmol) Aldimin ***AL1*** gegeben und die Mischung mittels eines Zentrifugalmischers (SpeedMixer™ DAC 150, FlackTek Inc.) innig vermengt. Nach 10 Minuten war die NCO-Bande im FT-IR-Spektrum (bei 2265 $cm^{-1}$) nicht mehr nachweisbar. Man erhielt eine klare, homogene und geruchlose Flüssigkeit mit einer Viskosität bei 20˚C von 89 Pa·s und einem Amin-Gehalt von 0.41 mmol $NH_2$/g.

**Beispiel 34 (Aldimin-haltige Verbindung *AP11)***

In einem Rundkolben wurden unter Stickstoffatmosphäre 5.85 g (0.052 mol NCO) Isophorondiisocyanat (Vestanat® IPDI, Degussa) vorgelegt. Unter Rühren wurden bei Raumtemperatur aus einem Eintropftrichter innerhalb von 5 Minuten 10.00 g (0.026 mol) Aldimin ***AL1*** zugegeben und die Mischung während 30 Minuten gerührt. Zum so erhaltenen klaren, farblosen Öl gab man bei Raumtemperatur 3.38 g (0.026 mol) 2-Hydroxyethylmethacrylat (HEMA; Bisomer® HEMA, Laporte). Man liess 10 Minuten rühren, gab dann 2 mg Dibutylzinndilaurat zu, erwärmte die Mischung auf 75°C und hielt sie solange bei dieser Temperatur, bis die Isocyanatbande im FT-IR-Spektrum (bei 2253 $cm^{-1}$) verschwunden war (1 Stunde). Man erhielt eine farblose, dickflüssige, klare und geruchlose Flüssigkeit, die einen Amin-Gehalt von 1.32 mmol $NH_2$/g aufwies. IR: 3334(N-H),2952,2923,2852,1719(C=O),1663sh(C=N),1636(C=C,C=O),1527,1459, 1377, 1364, 1341, 1296, 1234, 1164, 1060, 1044, 1017, 939, 891, 869, 814, 770, 721.

**Beispiel 35 (Aldimin-haltige Verbindung *AP12)***

In einem Rundkolben wurden unter Stickstoffatmosphäre 5.48 g (26.0 mmol NCO) m-Isopropenyl-$\alpha$,$\alpha$-dimethylbenzylisocyanat (m-TMI; Cytec) vorgelegt. Unter Rühren wurden bei Raumtemperatur aus einem Eintropftrichter innerhalb von 5 Minuten 10.00 g (26.0 mmol) Aldimin ***AL1*** zugegeben und die Mischung solange gerührt, bis die Isocyanatbande im FT-IR-Spektrum (bei 2255 $cm^{-1}$) verschwunden war (30 Minuten). Man erhielt eine farblose, dickflüssige, klare und geruchlose Flüssigkeit, die einen Amin-Gehalt von 1.66 mmol $NH_2$/g aufwies.
IR: 3361 (N-H), 2953sh, 2922, 2852, 1736 (C=O), 1689, 1658, 1646, 1600, 1578, 1523, 1483, 1465, 1457, 1440sh, 1417sh, 1375, 1361sh, 1346sh, 1302, 1241, 1218sh, 1162, 1111, 1051, 1015, 1003, 938, 886, 797, 764, 722, 695.

**Beispiel 36 (Aldimin-haltige Verbindung *AP13)***

In einem Rundkolben wurden unter Stickstoffatmosphäre eine Mischung von 2.57 g (8.7 mmol) Trimethylolpropan-triacrylat (TMPTA; SR-351, Sartomer) und 10.00 g (26.0 mmol) Aldimin ***AL1*** auf 90°C erwärmt und solange auf dieser Temperatur gehalten, bis die Acrylbande im FT-IR-Spektrum ($\delta_{C=C\text{-}H\ oop}$ bei 808 $cm^{-1}$) verschwunden war (3 Stunden). Man erhielt eine farblose, niedrigviskose, klare und geruchlose Flüssigkeit, die einen Amin-Gehalt von 4.06 mmol $NH_2$/g aufwies.
IR: 2952sh, 2922, 2851, 2795sh, 2771sh, 1736 (C=O), 1667 (C=N), 1464, 1419, 1392sh, 1375, 1345, 1300, 1248, 1163, 1120, 1054, 1032, 1009, 934, 876, 783, 722.

**Beispiel 37 (Aldimin-haltige Verbindung *AP14*)**

In einem Rundkolben wurden unter Stickstoffatmosphäre eine Mischung von 1.37 g (4.6 mmol) Trimethylolpropan-triacrylat (TMPTA; SR-351, Sartomer) und 10.00 g (13.9 mmol) Aldimin *AL3* auf 105°C erwärmt und solange auf dieser Temperatur gehalten, bis die Acrylbande im FT-IR-Spektrum ($\delta_{C=C\text{-}H\ oop}$ bei 808 $cm^{-1}$) verschwunden war (18 Stunden). Man erhielt eine gelbe, dickflüssige, klare und geruchlose Flüssigkeit, die einen Amin-Gehalt von 3.48 mmol $NH_2$/g aufwies.
IR: 2952sh, 2922, 2851, 1736 (C=O), 1667 (C=N), 1465, 1418, 1392, 1374, 1347, 1300sh, 1246, 1163, 1113, 1054, 1057, 1017, 999, 935, 879, 781, 722.

**Beispiel 38 (Aldimin-haltige Verbindung *AP15)***

In einem Rundkolben wurden unter Stickstoffatmosphäre eine Mischung von 9.56 g (52.0 mmol Epoxy) Bisphenol-A-diglycidylether (DGEBA oder BPADGE; Araldite® GY-250, Huntsman) und 20.00 g (52.0 mmol) Aldimin ***AL1*** auf 70°C erwärmt und solange auf dieser Temperatur gehalten, bis die Epoxybanden im FT-IR-Spektrum ($\nu_{C\text{-}Oasy}$ bei 914 und 861 $cm^{-1}$) verschwunden waren (16 Stunden). Man erhielt eine farblose, hochviskose, klare und geruchlose Flüssigkeit, die einen Amin-Gehalt von 3.50 mmol $NH_2$/g aufwies. IR: 3420 (O-H), 3034, 2922, 2851,2064,1884,1736 (C=O),1667(C=N),1607,1580,1509, 1463,1417,1375,1297,1248,1180,1157,1108,1084,1038, 933, 883, 827, 806, 767, 722.

<u>Einkomponentige Kunststoffvorläufer enthaltend Additionsprodukte der Aldimine der Formel (I)</u>

**Beispiele 39 bis 45 und Beispiel 46 (Vergleich)**

Für jedes Beispiel wurden 100.0 g Polyurethanpolymer ***PP1***, dessen Herstellung nachfolgend beschrieben wird, in einen Polypropylenbecher mit Schraubverschluss eingewogen und unter trockenen Stickstoff gestellt. Dazu

wurden 0.3 g einer Salicylsäurelösung (5 Gewichts-% in Dioctyladipat) gegeben sowie das der Tabelle 1 aufgeführte Aldimin der Formel (1) in der angegebenen Menge gegeben, die Mischung mittels eines Zentrifugalmischers (SpeedMixer™ DAC 150, FlackTek Inc.) innig vermengt, unmittelbar danach in eine innenlackierte Aluminiumtube abgefüllt und diese luftdicht verschlossen. Die Menge an zugegebenem Aldimin der Formel (I) entspricht für alle Beispiele einem Verhältnis von 1.0 / 0.7 zwischen den Isocyanatgruppen im Polyurethanpolymer und der Summe der Reaktivgruppen (Aldiminogruppen plus Amino-oder Hydroxylgruppen) im Aldimin.

Das Polyurethanpolymer ***PP1*** wurde wie folgt hergestellt:
1300 g Polyoxypropylen-Diol (Acclaim® 4200 N, Bayer; OH-Zahl 28.5 mg KOH/g), 2600 g Polyoxypropylenpolyoxyethylen-Triol (Caradol® MD34-02, Shell; OH-Zahl 35.0 mg KOH/g), 605 g 4,4'-Methylendiphenyldiisocyanat (MDI; Desmodur® 44 MC L, Bayer) und 500 g Diisodecylphthalat (DIDP; Palatinol® Z, BASF) wurden bei 80°C zu einem NCO-terminierten Polyurethanpolymeren mit einem titrimetrisch bestimmten Gehalt an freien Isocyanat-Gruppen von 2.07 Gewichts-% und einer Viskosität bei 20°C von 48 Pa·s umgesetzt.

Der so erhaltene einkomponentige Kunststoffvorläufer wurde auf Lagerstabilität, Hautbildungszeit, Blasenbildung, Geruch und mechanische Eigenschaften nach Aushärtung geprüft.
Die **Lagerstabilität** wurde über die Veränderung der Viskosität während der Lagerung in der Wärme bestimmt. Dazu wurde der Kunststoffvorläufer in der verschlossenen Tube im Ofen bei 60°C gelagert und seine Viskosität ein erstes Mal nach 12 Stunden, ein zweites Mal nach 7 Tagen Lagerdauer gemessen. Die Lagerstabilität ergibt sich aus der prozentualen Zunahme des zweiten Viskositätswerts gegenüber dem ersten.

Die Ergebnisse der Prüfungen sind in der Tabelle 1 aufgeführt. Aus Tabelle 1 ist ersichtlich, dass die einkomponentigen Kunststoffvorläufer der Beispiele 39 bis 45, welche Aldimin-haltige Verbindungen **AV** enthalten, die in situ hergestellte Additionsprodukte aus den erfindungsgemässen Aldiminen ***AL1*** bis ***AL7*** der Formel (I) der Beispiele 1 bis 7 und dem Polyurethanpolymer ***PP1*** darstellen, eine gegenüber dem einkomponentigen Kunststoffvorläufer des Referenzbeispiels, der kein Aldimin enthält, eine vergleichbar grosse Viskositätserhöhung nach Lagerung aufweisen. Dem gegenüber steigt die Viskosität des einkomponentigen Kunststoffvorläufers des Vergleichsbeispiels 46, welcher eine Aldimin-haltige Verbindung nach dem Stand der Technik enthält, die das in situ hergestellte Additionsprodukt aus dem Aldimin ***AL8*** des Vergleichsbeispiels 8 mit dem Polyurethanpolymer ***PP1*** darstellt, deutlich stärker an.

Tabelle 1: Zusammensetzung und Lagerstabilität von einkomponentigen Kunststoffvorläufern.

| Beispiel | Aldimin der Formel (I) | Aldimin-zugabe [g] | $\frac{[NCO]}{[[OH]+[NH]]}$ | Viskositäts-zunahme [%][a] |
|---|---|---|---|---|
| **(Ref)[b]** | - | - | - | 16 |
| **39** | ***AL1*** | 6.6 | 1.0/0.7 | 18 |
| **40** | ***AL2*** | 7.9 | 1.0/0.7 | 26 |
| **41** | ***AL3*** | 8.3 | 1.0/0.7 | 18 |
| **42** | ***AL4*** | 9.4 | 1.0/0.7 | 25 |
| **43** | ***AL5*** | 8.5 | 1.0/0.7 | 27 |
| **44** | ***AL6*** | 7.8 | 1.0/0.7 | 13 |
| **45** | ***AL7*** | 11.2 | 1.0/0.7 | 23 |
| **46 (Vergleich)** | ***AL8*** | 4.9 | 1.0 / 0.7 | 42 |

[a] = (Viskosität nach 7 d / Viskosität nach 12 Std. - 1) × 100%.
[b] Referenzbeispiel ohne Aldimin.

Zur Bestimmung der **Hautbildungszeit** (Zeit bis zur Klebefreiheit, "tack-free time") wurde ein kleiner Teil des während 12 Stunden bei 60°C gelagerten, raumtemperaturwarmen Kunststoffvorläufers in einer Schichtdicke von 3 mm auf Pappkarton aufgetragen und bei 23°C und 50% relativer Luftfeuchtigkeit die Zeit bestimmt, die es dauerte, bis beim leichten Antippen der Polymeroberfläche mittels einer Pipette aus LDPE erstmals keine Polymerrückstände auf der Pipette mehr zurückblieben.

Zur Bestimmung der **mechanischen Eigenschaften** nach Aushärtung wurde ein weiterer Teil des während 12 Stunden bei 60°C gelagerten Kunststoffvorläufers als Film von ca. 2 mm Dicke in ein mit PTFE beschichtetes Blech gegossen, worauf man den Film während 7 Tagen bei 23°C und 50% relativer Luftfeuchtigkeit zu einem elastischen

Kunststoff aushärten liess. Der so hergestellte Kunststofffilm wurde gemäss DIN EN 53504 auf **Zugfestigkeit**, Bruchdehnung und **E-Modul** geprüft (Zuggeschwindigkeit: 200 mm/min).

Qualitativ beurteilt wurden ausserdem die **Blasenbildung** (anhand der Menge Blasen, die während der Aushärtung des Films auftraten) sowie der **Geruch** (durch Riechen mit der Nase in einem Abstand von 10 cm, zuerst am frisch gegossenen Film und wieder am vollständig ausgehärteten Film).

Die Ergebnisse der Prüfungen sind in der Tabelle 2 aufgeführt.

Tabelle 2: Eigenschaften während und nach der Aushärtung von einkomponentigen Kunststoffvorläufern.

| Beispiel | **39** | **40** | **41** | **42** | **43** | **44** | **45** | **46 (Vgl)** |
|---|---|---|---|---|---|---|---|---|
| Hautbildung (min.) | 35 | 45 | 35 | 50 | 40 | 35 | 45 | 90 |
| Blasenbildung | keine | keine | keine | keine | keine | keine | keine | einige |
| Zugfestigkeit (MPa) | 0.8 | 1.0 | 0.8 | 0.7 | 0.7 | 0.7 | 1.0 | 1.0 |
| Bruchdehnung (%) | 180 | 200 | 60 | 70 | 80 | 130 | 220 | 240 |
| E-Modul (MPa)[a] | 1.3 | 1.4 | 2.3 | 1.7 | 1.6 | 1.3 | 1.3 | 1.1 |
| Geruch | kein | kein | kein | kein | kein | kein | kein | stark |
| [a] bei 0.5-5.0% Dehnung. | | | | | | | | |

Aus Tabelle 2 ist ersichtlich, dass die Kunststoffvorläufer der Beispiele 39 bis 45, welche jeweils ein in situ hergestelltes Additionsprodukt der erfindungsgemässen Aldimine ***AL1*** bis ***AL7*** enthalten, rasch und ohne Blasenbildung aushärten, geruchsfrei sind und im ausgehärteten Zustand gute mechanische Eigenschaften besitzen. Demgegenüber härtet der Kunststoffvorläufer des Vergleichsbeispiels 46, welcher ein in situ hergestelltes Additionsprodukt des Aldimins ***AL8*** nach dem Stand der Technik enthält, langsamer und unter partieller Blasenbildung aus und weist einen starken Geruch auf.

<u>Zweikomponentige Kunststoffvorläufer enthaltend Aldimine der Formel (I)</u>

**Beispiele 47 bis 50 und Beispiel 51 (Vergleich)**

Für jedes Beispiel wurden die in Tabelle 3 angegebenen Gewichtsteile der Bestandteile in der jeweiligen Komponente **L1** ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) innig zu einer homogenen Creme vermengt. Dazu wurde für jedes Beispiel die in Tabelle 3 angegebenen Gewichtsteile an PMDI als Komponente **L2** zugegeben und erneut innig gemischt (30 sec. bei 3000 U/min). Das Verhältnis zwischen den Isocyanatgruppen der Komponente **L2** und der Summe der Reaktivgruppen (Hydroxyl-, Amino- und Aldiminogruppen) der Komponente **L1** beträgt für alle Beispiele 1.1 /1.0.

Tabelle 3: Zusammensetzung von zweikomponentigen Kunststoffvorläufern.

| Beispiel | **47** | **48** | **49** | **50** | **51 (Vgl)** |
|---|---|---|---|---|---|
| **Komponente L1:** | | | | | |
| Ricinusöl[a] | 19.4 | 19.9 | 19.9 | 15.9 | 18.5 |
| Dimerfettsäurepolyol[b] | 11.0 | 16.0 | 16.0 | 16.0 | 14.8 |
| Triol[c] | 5.5 | - | - | - | 3.7 |
| Aldimin der Formel (I) | ***AL1,*** 4.0 | ***AL19,*** 4.0 | ***AL20,*** 4.0 | ***AL23,*** 8.0 | - |
| Weichmacher[d] | - | - | - | - | 3.0 |
| Säurekatalysator[e] | 0.1 | 0.1 | 0.1 | 0.1 | - |
| Kreide[f] | 60.0 | 60.0 | 60.0 | 60.0 | 60.0 |

(fortgesetzt)

| **Komponente L2:** | | | | | |
|---|---|---|---|---|---|
| PMDI[g] | 26.3 | 20.9 | 25.9 | 25.9 | 22.1 |

[a] Fluka;OH-Zahl=165 mg KOH/g. [b] Sovermol® 908,Cognis;OH-Zahl=200 mg KOH/g. [c] Desmophen® 4011 T, Bayer; OH-Zahl = 550 mg KOH/g.
[d] Diisodecylphthalat, Palatinol® Z, BASF. [e] 5 Gew.-% Salicylsäure in Dioctyladipat. [f] Omyacarb® 5-GU, Omya. [g] Desmodur® VKS 20 F, Bayer; NCO-Gehalt = 30.0 Gewichts-%.

Die so erhaltenen gemischten zweikomponentigen Kunststoffvorläufer wurden auf Thixotropie, Aushärtegeschwindigkeit und mechanische Eigenschaften geprüft. Die **Thixotropie** wurde qualitativ anhand des Fliessverhaltens des gemischten zweikomponentigen Kunststoffvorläufers, unmittelbar nach dem Mischen der Komponenten **L1** und **L2,** auf einer LDPE-Unterlage beurteilt. Keine Thixotropie bedeutet starkes Verfliessen, während starke Thixotropie kein Verfliessen bedeutet (Strukturviskosität). Hinweise zur Aushärtegeschwindigkeit wurden zum einen durch Messen der **Zeit bis zur Klebefreiheit** ("tack-free time") des gemischten zweikomponentigen Kunststoffvorläufers, unmittelbar nach dem Mischen der Komponenten **L1** und **L2**, erhalten. Die Messmethode entspricht jener Methode, wie sie bei Beispiel 39 zur Messung der Hautbildungszeit für einkomponentige Kunststoffvorläufer beschrieben wurde. Zum anderen wurde der weitere Verlauf der Aushärtung durch periodisches Messen der **Shore D-Härte** nach DIN EN 53505 verfolgt. Zur Prüfung der mechanischen Eigenschaften wurde der gemischte zweikomponentige Kunststoffvorläufer in dünner Schicht in eine plane PTFE-Form gegossen bzw., bei thixotropen Mischungen, gerakelt, der Film während 7 d im Normklima ausgehärtet und nach DIN EN 53504 auf **Zugfestigkeit**, Bruchdehnung und **E-Modul** (Zuggeschwindigkeit: 10 mm/min) geprüft. In qualitativer Weise beurteilt wurde ausserdem die **Blasenbildung** (anhand der Menge Blasen, die während der Aushärtung des Films auftraten).

Die Ergebnisse dieser Prüfungen sind in Tabelle 4 aufgeführt.

Tabelle 4: Eigenschaften von gemischten zweikomponentigen Kunststoffvorläufern.

| Beispiel | **47** | **48** | **49** | **50** | **51 (Vgl)** |
|---|---|---|---|---|---|
| Thixotropie | keine | Wenig | Mittel | stark | keine |
| Klebefreiheit (min.)[a] | 40 | 37 | 16 | 18 | 85 |
| Shore D nach 1 Tag | 52 | 50 | 45 | 41 | 35 |
| Shore D nach 3 Tagen | 68 | 67 | 66 | 61 | 52 |
| Shore D nach 7 Tagen | 75 | 72 | 66 | 65 | 66 |
| Shore D getempert[b] | 83 | 84 | 81 | 77 | 85 |
| Zugfestigkeit (MPa) | 9.2 | 9.0 | 9.0 | 7.6 | 7.6 |
| Bruchdehnung (%) | 60 | 60 | 60 | 60 | 60 |
| E-Modul (MPa)[c] | 58 | 60 | 39 | 28 | 45 |
| Blasenbildung | keine | Keine | Keine | keine | viele |

[a] Zeit bis zur Klebefreiheit (tack-free time) in Minuten.
[b] 4 h bei 105°C der während 7 Tagen im Normklima ausgehärteten Prüfkörper.
[c] bei 0.5-5.0% Dehnung.

Aus der Tabelle 4 ist ersichtlich, dass die gemischten zweikomponentigen Kunststoffvorläufer der Beispiele 47 bis 50, welche erfindungsgemässe Aldimine der Formel (I) enthalten, rasch aushärten, dabei trotz ungetrockneten Bestandteilen keine Blasen bilden und im ausgehärteten Zustand gute mechanische Eigenschaften besitzen. Die Beispiele 48 bis 50, welche Aldimine der Formel (I) mit mehr als einer sekundären Aminogruppe enthalten, zeigen zusätzlich thixotropes Verhalten. Demgegenüber härtet der gemischte zweikomponentige Kunststoffvorläufer des Vergleichsbeispiels 51 nach dem Stand der Technik ohne Aldimin langsamer aus und zeigt eine starke Blasenbildung.

## Patentansprüche

**1.** Aldimin der Formel (I)

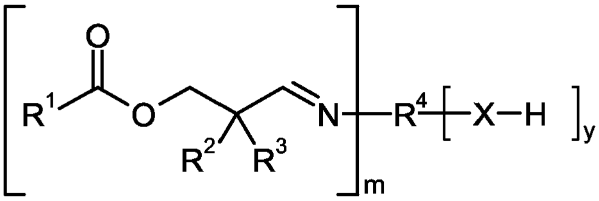

(I)

wobei

m für eine ganze Zahl von 1 bis 4 steht und
y für eine ganze Zahl von 1 bis 4 steht,
mit der Massgabe, dass m+y = 2 bis 5 ist;
und wobei
$R^1$ entweder
für einen einwertigen Kohlenwasserstoffrest mit 6 bis 30 C-Atomen, welcher gegebenenfalls mindestens ein Heteroatom, insbesondere in Form von Ether-Sauerstoff, aufweist, steht,
oder
für einen Substituenten der Formel (II) steht

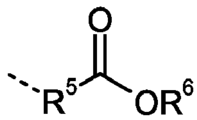

(II)

wobei
$R^5$ für einen zweiwertigen Kohlenwasserstoffrest mit 2 bis 20 C-Atomen, welcher gegebenenfalls mindestens ein Heteroatom, insbesondere in Form von Ether-Sauerstoff, aufweist, steht,
und
$R^6$ für einen einwertigen Kohlenwasserstoffrest mit 1 bis 20 C-Atomen steht;
und wobei
$R^2$ und $R^3$ entweder,
unabhängig voneinander, je für einen einwertigen
Kohlenwasserstoffrest mit 1 bis 12 C-Atomen stehen;
oder
zusammen einen zweiwertigen Kohlenwasserstoffrest mit 4 bis 20 C-Atomen bilden, der Teil eines, gegebenenfalls substituierten, carbocyclischen Rings mit 5 bis 8, bevorzugt 6, C-Atomen ist;
und wobei
$R^4$ für einen (m+y)-wertigen Kohlenwasserstoffrest mit 2 bis 12 C-Atomen, welcher gegebenenfalls mindestens ein Heteroatom, insbesondere in Form von Ether-Sauerstoff oder einem tertiären Amin-Stickstoff, enthält, steht;
und wobei
X für O, S oder N-$R^7$ steht,
wobei
$R^7$ entweder
für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureestergruppe aufweist, steht,
oder
für einen Substituenten der Formel (III) steht

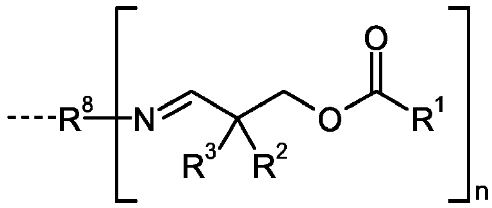 (III),

wobei
n für eine ganze Zahl von 1 bis 10'000 steht,
$R^8$ für einen (n+1)-wertigen Kohlenwasserstoffrest, welcher gegebenenfalls Heteroatome, insbesondere in Form von Ether-Sauerstoff oder tertiärem Amin-Stickstoff, und gegebenenfalls aktiven Wasserstoff in Form von Hydroxylgruppen, sekundären Aminogruppen oder Mercaptogruppen enthält, steht.

**2.** Aldimin gemäss Anspruch 1, **dadurch gekennzeichnet, dass** y=1 ist.

**3.** Aldimin gemäss Anspruch 1 oder 2, **dadurch gekennzeichnet, dass** $R^2$ und $R^3$ gleich sind und insbesondere je für eine Methylgruppe stehen.

**4.** Aldimin gemäss einem der vorhergehenden Ansprüche, **dadurch gekennzeichnet, dass** m+y = 2 oder 3, insbesondere 2, ist.

**5.** Aldimin gemäss einem der vorhergehenden Ansprüche, **dadurch gekennzeichnet, dass** X für N-$R^7$ steht und $R^7$ für entweder für einen einwertigen Kohlenwasserstoffrest mit 1 bis 20 C-Atomen, oder für einen einwertigen Kohlenwasserstoffrest der Formel (IX) oder (IX') steht

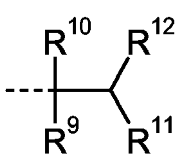 (IX)

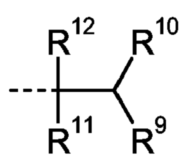 (IX')

wobei $R^9$ für einen Rest, welcher ausgewählt ist aus der Gruppe bestehend aus $-COOR^{13}$, -CN, $-NO_2$, $-PO(OR^{13})_2$, $-SO_2R^{13}$ und $-SO_2OR^{13}$, steht; $R^{10}$ für ein Wasserstoffatom oder einen Rest ausgewählt aus der Gruppe bestehend aus $-R^{13}$, $-COOR^{13}$ und $-CH_2COOR^{13}$ steht und
$R^{11}$ und $R^{12}$ unabhängig voneinander für ein Wasserstoffatom oder einen Rest ausgewählt aus der Gruppe bestehend aus $-R^{13}$, $-COOR^{13}$ und -CN steht, wobei $R^{13}$ für einen einwertigen Kohlenwasserstoffrest mit 1 bis 20 C-Atomen steht.

**6.** Aldimin gemäss einem der vorhergehenden Ansprüche, **dadurch gekennzeichnet, dass** X für O oder S steht.

**7.** Aldimin der Formel (X),

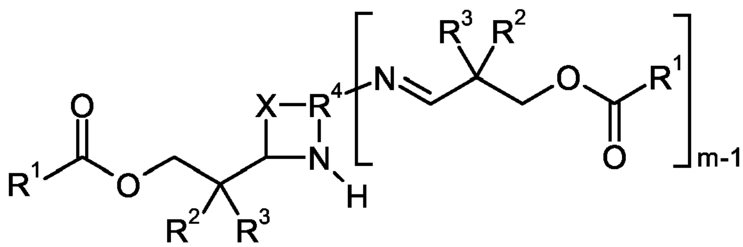 (X)

**dadurch gekennzeichnet, dass** es durch eine Cyclisierungsreaktion eines Aldimins der Formel (I) gemäss einem der Ansprüche 1-6 erhalten wird.

**8.** Aldimin-haltige Verbindung, **dadurch gekennzeichnet, dass** diese durch die Umsetzung eines Aldimins der Formel (I) gemäss einem der Ansprüche 2 - 7 mit einer Verbindung **D**, die mehr als eine Reaktivgruppe trägt, welche mit der Gruppe XH Additionsreaktionen eingehen kann, erhalten wird.

**9.** Aldimin-haltige Verbindung gemäss Anspruch 8, **dadurch gekennzeichnet, dass** die Verbindung **D** ein Polyisocyanat ist.

**10.** Aldimin-haltige Verbindung gemäss Anspruch 8, **dadurch gekennzeichnet, dass** die Verbindung **D** ein Polyepoxid ist.

**11.** Aldimin-haltige Verbindung gemäss einem der Ansprüche 8-10, **dadurch gekennzeichnet, dass** das Aldimin der Formel (I) in einem Verhältnis von einem Molequivalent aktiven Wasserstoff des Aldimins auf ein Molequivalent Reaktivgruppen der Verbindung **D** eingesetzt wird.

**12.** Aldimin-haltige Verbindung gemäss einem der Ansprüche 8 - 10, **dadurch gekennzeichnet, dass** das Aldimin der Formel (I) in einem Verhältnis von weniger als einem Molequivalent aktiven Wasserstoff des Aldimins auf ein Molequivalent Reaktivgruppen der Verbindung **D** eingesetzt wird.

**13.** Verwendung eines Aldimins der Formel (I) gemäss einem der Ansprüche 1-7 oder einer Aldimin-haltigen Verbindung gemäss einem der Ansprüche 8 - 12 als Quelle eines Aldehyds der Formel (IV)

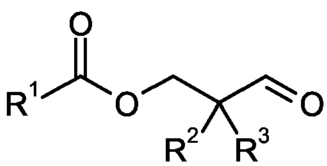 (IV)

**14.** Verwendung eines Aldimins der Formel (I) gemäss einem der Ansprüche 1-7 oder einer Aldimin-haltigen Verbindung gemäss einem der Ansprüche 8 - 12 als Quelle eines Amins der Formel $[H_2N]_m$-$R^4$-[XH]y.

**15.** Verfahren der Hydrolyse eines Aldimins der Formel (I) gemäss einem der Ansprüche 1 - 7 oder einer Aldimin-haltigen Verbindung gemäss einem der Ansprüche 8-12.

**16.** Verwendung eines Aldimins der Formel (I) gemäss einem der Ansprüche 1-7 oder einer Aldimin-haltigen Verbindung gemäss einem der Ansprüche 8 - 12 als geschützter Vernetzer für einen Kunststoffvorläufer.

**17.** Zusammensetzung enthaltend ein Aldimin der Formel (I) gemäss einem der Ansprüche 1 - 7 oder eine Aldimin-haltigen Verbindung gemäss einem der Ansprüche 8 - 12.

Europäisches Patentamt

# EUROPÄISCHER RECHERCHENBERICHT

Nummer der Anmeldung
EP 05 10 9110

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 384 735 A (SIKA TECHNOLOGY AG) 28. Januar 2004 (2004-01-28) * Absätze [0046], [0049]; Ansprüche 1,9-13; Verbindung V * | 1-6,8-17 | INV. C07C251/08 C07D265/06 C07D277/04 |
| A | EP 1 544 204 A (SIKA TECHNOLOGY AG) 22. Juni 2005 (2005-06-22) * das ganze Dokument * | 1-17 | |
| A | EP 0 469 751 A (MITSUI TOATSU CHEMICALS, INC) 5. Februar 1992 (1992-02-05) * das ganze Dokument * | 1-17 | |
| A | US 4 469 831 A (BUELTJER ET AL) 4. September 1984 (1984-09-04) * das ganze Dokument * | 1-17 | |
| D,A | US 6 136 942 A (PFENNINGER ET AL) 24. Oktober 2000 (2000-10-24) * das ganze Dokument * | 1-17 | RECHERCHIERTE SACHGEBIETE (IPC) |
| D,A | US 4 224 417 A (HAJEK ET AL) 23. September 1980 (1980-09-23) * das ganze Dokument * | 1-17 | C07C C07D |

Der vorliegende Recherchenbericht wurde für alle Patentansprüche erstellt

| Recherchenort | Abschlußdatum der Recherche | Prüfer |
|---|---|---|
| München | 21. Juni 2006 | Österle, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

2

EPO FORM 1503 03.82 (P04C03)

Europäisches
Patentamt

Nummer der Anmeldung
EP 05 10 9110

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

Europäisches Patentamt

**MANGELNDE EINHEITLICHKEIT DER ERFINDUNG ERGÄNZUNGSBLATT B**

Nummer der Anmeldung
EP 05 10 9110

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-6, 8-12, 13-17 (Teil)

   Aldimin der Formel (I) (Ansprüche 1-6), Aldimin-haltige Verbindung (Ansprüche 8-12), Verwendung des Aldimins der Formel (I) gemäss einem der Ansprüche 1-6 (Ansprüche 13, 14, 16 ), Verfahren der Hydrolyse eines Aldimins der Formel (I) gemäss einem der Ansprüche 1-6 (Anspruch 15), Zusammensetzung enthaltend ein Aldimin der Formel (I) (Anspruch 17)

   ---

2. Ansprüche: 7, 13-17 (Teil)

   Aldimin der Formel (II) (Anspruch 7), Verwendung des Aldimins der Formel (II) gemäss einem des Anspruchs 7 (Ansprüche 13, 14, 16 ), Verfahren der Hydrolyse eines Aldimins der Formel (II) gemäss des Anspruchs 7 (Anspruch 15), Zusammensetzung enthaltend ein Aldimin der Formel (II) (Anspruch 17)

   ---

## ANHANG ZUM EUROPÄISCHEN RECHERCHENBERICHT ÜBER DIE EUROPÄISCHE PATENTANMELDUNG NR. EP 05 10 9110

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-06-2006

| Im Recherchenbericht angeführtes Patentdokument | Datum der Veröffentlichung | Mitglied(er) der Patentfamilie | Datum der Veröffentlichung |
|---|---|---|---|
| EP 1384735 A | 28-01-2004 | AU 2003251462 A1 | 23-02-2004 |
| | | BR 0312965 A | 14-06-2005 |
| | | CA 2493600 A1 | 12-02-2004 |
| | | CN 1678653 A | 05-10-2005 |
| | | WO 2004013200 A1 | 12-02-2004 |
| | | JP 2005533916 T | 10-11-2005 |
| | | US 2006122352 A1 | 08-06-2006 |
| EP 1544204 A | 22-06-2005 | WO 2005058921 A2 | 30-06-2005 |
| EP 0469751 A | 05-02-1992 | DE 69109762 D1 | 22-06-1995 |
| | | DE 69109762 T2 | 21-12-1995 |
| | | KR 9706961 B1 | 01-05-1997 |
| | | US 5087661 A | 11-02-1992 |
| US 4469831 A | 04-09-1984 | KEINE | |
| US 6136942 A | 24-10-2000 | AT 274006 T | 15-09-2004 |
| | | CA 2267702 A1 | 02-10-1999 |
| | | DE 59910235 D1 | 23-09-2004 |
| | | EP 0947529 A1 | 06-10-1999 |
| | | ES 2227924 T3 | 01-04-2005 |
| | | JP 11322893 A | 26-11-1999 |
| US 4224417 A | 23-09-1980 | AT 353009 B | 25-10-1979 |
| | | AT 768576 A | 15-03-1979 |
| | | AU 504102 B2 | 04-10-1979 |
| | | AU 1876476 A | 27-04-1978 |
| | | BE 847333 A1 | 15-04-1977 |
| | | CA 1075397 A1 | 08-04-1980 |
| | | DE 2546536 A1 | 28-04-1977 |
| | | ES 452450 A1 | 01-11-1977 |
| | | FR 2328006 A1 | 13-05-1977 |
| | | GB 1531578 A | 08-11-1978 |
| | | IT 1069070 B | 25-03-1985 |
| | | JP 1195629 C | 12-03-1984 |
| | | JP 57185312 A | 15-11-1982 |
| | | JP 58030329 B | 28-06-1983 |
| | | JP 1164272 C | 26-08-1983 |
| | | JP 52050396 A | 22-04-1977 |
| | | JP 57057051 B | 02-12-1982 |
| | | SE 7611420 A | 18-04-1977 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4469831 A
- US 4853454 A
- US 5087661 A
- WO 2004013088 A1
- US 4224417 A
- US 3493543 A
- US 3554974 A
- US 4108842 A
- US 4404379 A
- US 6136942 A


### In der Beschreibung aufgeführte Nicht-Patentliteratur


- **HOUBEN-WEYL.** Methoden der organischen Chemie. vol. Xl/2, 73ff
- **HOUBEN-WEYL.** Methoden der organischen Chemie. vol. VIII, 516-528